# EUROPÄISCHE PATENTSCHRIFT

(11) **EP 4 084 304 B1**
(45) Veröffentlichungstag und Bekanntmachung des Hinweises auf die Patenterteilung: **14.08.2024**
(21) Anmeldenummer: 22168222.2
(22) Anmeldetag: 13.04.2022
(51) Int. Cl.: H02K 7/09, H02K 7/14, H02K 21/18, H02K 1/14, A61M 60/422, H02K 19/10, H02K 21/16

(54) **ELEKTROMAGNETISCHER DREHANTRIEB, ZENTRIFUGALPUMPE UND PUMPENEINHEIT**
ELECTROMAGNETIC ROTARY ACTUATOR, CENTRIFUGAL PUMP AND PUMP UNIT
ENTRAÎNEMENT ROTATIF ÉLECTROMAGNÉTIQUE, POMPE CENTRIFUGE ET ENSEMBLE POMPE

(30) Priorität: 26.04.2021 EP 21170398
(43) Veröffentlichungstag der Anmeldung: 02.11.2022
(73) Patentinhaber: Levitronix GmbH, 8048 Zürich (CH)
(72) Erfinder: Stettler, Marcel, 5600 Lenzburg (CH); Schneeberger, Thomas Dr., 3006 Bern (CH)
(74) Vertreter: IPS Irsch AG

(56) Entgegenhaltungen:
- EP-A1- 0 899 857
- EP-A1- 2 065 085
- EP-A1- 2 273 124
- EP-A1- 2 280 471
- EP-A1- 3 232 549
- EP-A1- 3 241 609
- EP-A1- 3 444 477
- EP-A1- 3 570 415
- US-A1- 2002 000 228

## Beschreibung

Die Erfindung betrifft einen elektromagnetischen Drehantrieb, eine Zentrifugalpumpe mit einem solchen elektromagnetischen Drehantrieb, sowie eine Pumpeneinheit für eine solche Zentrifugalpumpe gemäss dem Oberbegriff des unabhängigen Patentanspruchs der jeweiligen Kategorie.

Es sind elektromagnetische Drehantriebe bekannt, die nach dem Prinzip des lagerlosen Motors ausgestaltet sind und betrieben werden. Mit dem Begriff lagerloser Motor ist dabei ein elektromagnetischer Drehantrieb gemeint, bei welchem der Rotor vollkommen magnetisch bezüglich des Stators gelagert ist, wobei keine separaten magnetischen Lager vorgesehen sind. Der Stator ist dazu als Lager- und Antriebsstator ausgestaltet, der sowohl Stator des elektrischen Antriebs als auch Stator der magnetischen Lagerung ist. Mit den elektrischen Wicklungen des Stators lässt sich ein magnetisches Drehfeld erzeugen, welches zum einen ein Drehmoment auf den Rotor ausübt, das dessen Rotation um eine Solldrehachse bewirkt, und welches zum anderen eine beliebig einstellbare Querkraft auf den Rotor ausübt, sodass dessen radiale Position aktiv steuerbar bzw. regelbar ist. Somit sind drei Freiheitsgrade des Rotors aktiv regelbar, nämlich seine Rotation sowie seine radiale Position (zwei Freiheitsgrade). Bezüglich dreier weiterer Freiheitsgrade, nämlich seiner Position in axialer Richtung und Verkippungen bezüglich der zur Solldrehachse senkrechten radialen Ebene (zwei Freiheitsgrade), ist der Rotor passiv magnetisch, das heisst nicht ansteuerbar, durch Reluktanzkräfte gelagert bzw. stabilisiert. Das Nichtvorhandensein eines separaten magnetischen Lagers bei vollständiger magnetischer Lagerung des Rotors ist die Eigenschaft, welcher der lagerlose Motor seinen Namen verdankt. In dem Lager- und Antriebsstator lässt sich die Lagerfunktion nicht von der Antriebsfunktion separieren.

Ein solcher lagerloser Motor hat sich in einer Vielzahl von Anwendungen bewährt. Aufgrund der Abwesenheit von mechanischen Lagern eignet sich der lagerlose Motor insbesondere für Pump-, Misch- oder Rührvorrichtungen, mit denen sehr empfindliche Substanzen gefördert werden, beispielsweise Blutpumpen, oder bei denen sehr hohe Anforderungen an die Reinheit gestellt werden, beispielsweise in der pharmazeutischen Industrie oder in der biotechnologischen Industrie, oder mit denen abrasive oder aggressive Substanzen gefördert werden, welche mechanische Lager sehr schnell zerstören würden, beispielsweise Pumpen oder Mischer für Slurry in der Halbleiterindustrie.

Ein weiterer Vorteil des Prinzips des lagerlosen Motors ergibt sich bei der Ausgestaltung des Rotors als Integralrotor, der sowohl der Rotor des elektromagnetischen Drehantriebs ist, als auch der Rotor der Pumpe. Neben der berührungslosen magnetischen Lagerung resultiert hier der Vorteil einer sehr kompakten und platzsparenden Ausgestaltung.

Zudem erlaubt das Prinzip des lagerlosen Motors auch Ausgestaltungen, beispielsweise von Zentrifugalpumpen, bei denen der Rotor sehr leicht vom Stator trennbar ist. Dies ist ein sehr grosser Vorteil, weil damit beispielsweise der Rotor bzw. die Pumpeneinheit, welche den Rotor umfasst, als Einmalteil für den Einmalgebrauch ausgestaltet werden kann. Solche Einmalanwendungen ersetzen heute häufig Prozesse, bei denen früher aufgrund der sehr hohen Reinheitsanforderungen alle diejenigen Komponenten, welche im Prozess mit den zu behandelnden Substanzen in Kontakt kommen, aufwändig gereinigt und sterilisiert werden müssen, beispielsweise mittels Dampfsterilisierung. Bei der Ausgestaltung für den Einmalgebrauch werden diejenigen Komponenten, welche mit den zu behandelnden Substanzen in Kontakt kommen, nur genau einmal verwendet und dann bei der nächsten Anwendung durch neue, das heisst ungebrauchte, Einmalteile ersetzt.

Als Beispiele seien hier die Pharmaindustrie und die biotechnologische Industrie genannt. Hier werden häufig Lösungen und Suspensionen hergestellt, die eine sorgfältige und schonende Förderung von Substanzen verlangen.

In der Pharmaindustrie müssen beispielsweise bei der Herstellung von pharmazeutisch wirksamen Substanzen höchste Ansprüche an die Reinheit gestellt werden, oft müssen die mit den Substanzen in Kontakt kommenden Komponenten sogar steril sein. Ähnliche Anforderungen ergeben sich auch in der Biotechnologie, beispielsweise bei der Herstellung, Behandlung oder Züchtung von biologischen Substanzen, Zellen oder Mikroorganismen, wo ein extrem hohes Mass an Reinheit gewährleistet sein muss, um die Brauchbarkeit des hergestellten Produkts nicht zu gefährden. Als ein weiteres Beispiel seien hier Bioreaktoren genannt, in denen beispielsweise biologische Substitute für Gewebe oder spezielle Zellen oder andere sehr empfindliche Substanzen gezüchtet werden. Auch hier benötigt man Zentrifugalpumpen, um beispielsweise eine kontinuierliche Durchmischung der Nährflüssigkeit beziehungsweise deren kontinuierliche Zirkulation im Mischbehälter zu gewährleisten. Dabei muss eine sehr hohe Reinheit gewährleistet sein, um die Substanzen oder die erzeugten Produkte vor Kontaminationen zu schützen. Ein weiteres Anwendungsbeispiel sind Blutpumpen, bei denen natürlich höchste Anforderungen an die Reinheit und zudem an die schonende Behandlung insbesondere der roten Blutkörperchen gestellt werden.

Bei solchen Anwendungen, bei denen eine Zentrifugalpumpe für den Einmalgebrauch ausgelegt ist, setzt sich die Zentrifugalpumpe typischerweise aus einer Einmalvorrichtung und einer wiederverwendbaren Vorrichtung zusammen. Dabei umfasst die Einmalvorrichtung diejenigen Komponenten, die mit den Substanzen in Kontakt kommen, und die als Einmal (single-use) -Teile für den Einmalgebrauch ausgestaltet sind. Dies ist beispielsweise die Pumpeneinheit mit dem Pumpengehäuse und dem darin angeordneten Rotor, welcher das Flügelrad der Zentrifugalpumpe bildet. Die wiederverwendbare Vorrichtung umfasst diejenigen Komponenten, die dauerhaft also mehrfach verwendet werden, beispielsweise den Stator des elektromagnetischen Drehantriebs.

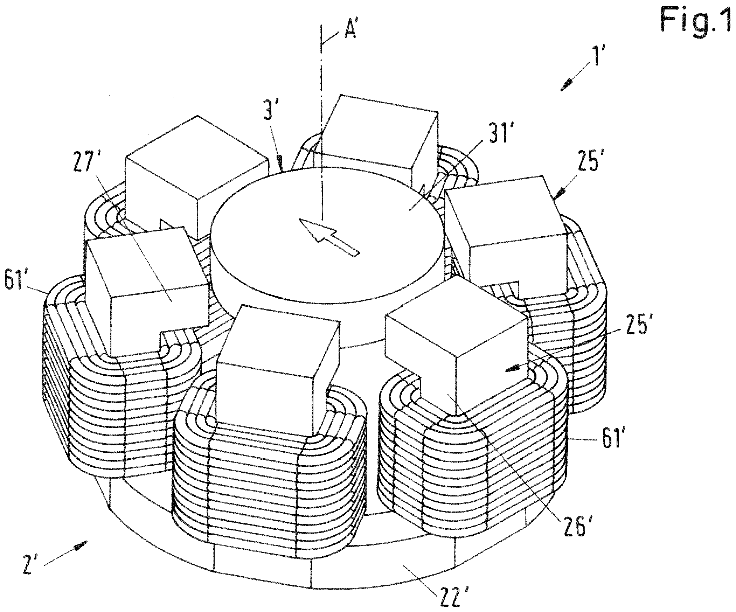

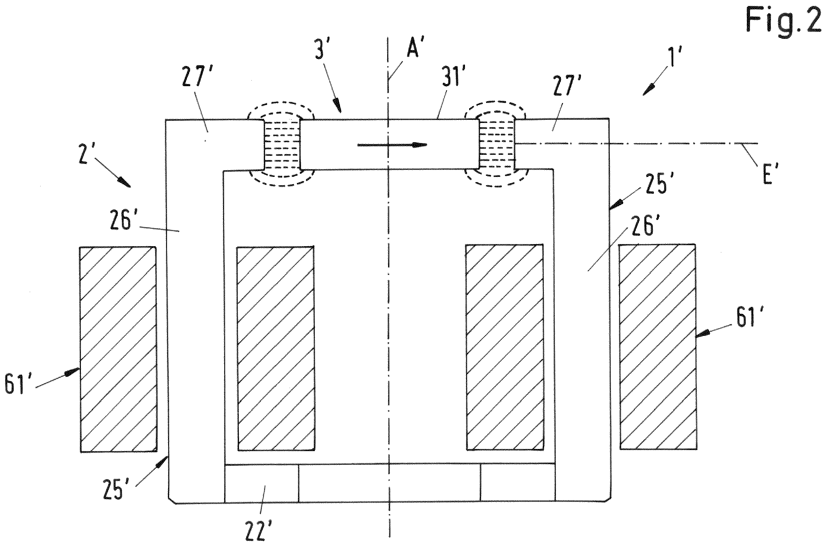

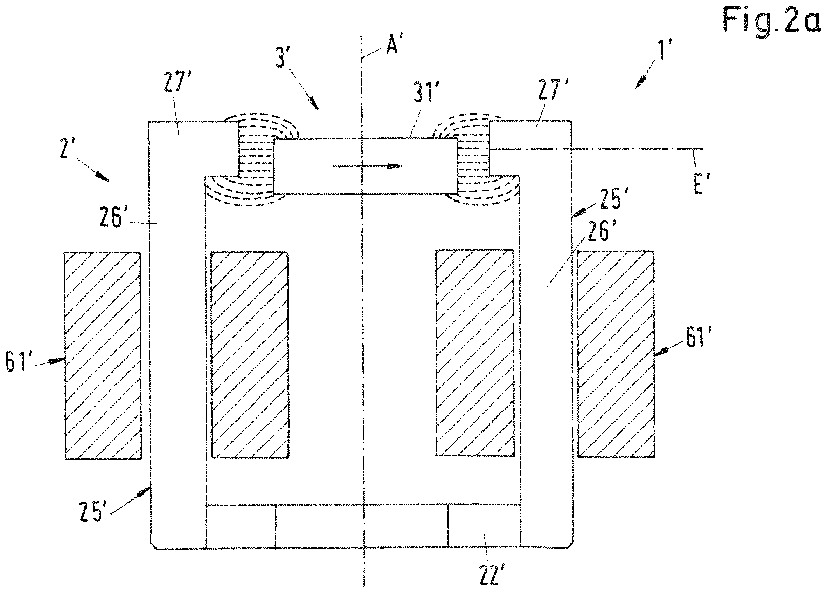

Eine vorteilhafte und an sich bekannte Ausführungsform eines elektromagnetischen Drehantriebs, der nach dem Prinzip des lagerlosen Motors ausgestaltet werden kann, ist die Ausführung als Tempelmotor, die beispielsweise in der EP-A-3 232 549 offenbart wird. Auf die Ausgestaltung als Tempelmotor bezieht sich auch die vorliegende Erfindung. In Fig. 1 ist in einer perspektivischen Darstellung eine Ausgestaltung eines Tempelmotors zu sehen, die vom Stand der Technik bekannt ist. Zum besseren Verständnis ist in Fig. 2 noch eine schematischere Darstellung des Tempelmotors in einem Schnitt in axialer Richtung gezeigt, wobei in Fig. 2 zusätzlich die Feldlinien des magnetischen Flusses durch die strichlierten Linien veranschaulicht sind. Ferner zeigt Fig. 2a in einer zu Fig. 2 analogen Darstellung den Tempelmotor für den Fall, dass der Rotor bezüglich der axialen Richtung ausgelenkt ist.

Um anzuzeigen, dass es sich bei der Darstellung in Fig. 1, in Fig. 2 und in Fig. 2a um eine Vorrichtung aus dem Stand der Technik handelt, sind hier die Bezugszeichen jeweils mit einem Hochkomma bzw. mit einem Strich versehen. Der Tempelmotor ist gesamthaft mit dem Bezugszeichen 1' bezeichnet.

Das Charakteristische eines Tempelmotors ist es, dass der Stator 2' eine Mehrzahl von Spulenkernen 25' aufweist, von denen jeder einen Längsschenkel 26` umfasst, der sich parallel zur axialen Richtung A' erstreckt. Mit der axialen Richtung A' ist dabei diejenige Richtung gemeint, welche durch die Solldrehachse des Rotors 3' definiert ist, also die Drehachse, um welche der Rotor 3' im Betriebszustand rotiert, wenn er in der radialen Ebene E', die senkrecht zur axialen Richtung A' liegt, bezüglich des Stators 2' in einer zentrierten und unverkippten Position ist. Jeder Längsschenkel 26' erstreckt sich von einem ersten, darstellungsgemäss unteren, Ende in axialer Richtung A' bis zu einem zweiten, darstellungsgemäss oberen Ende. Jeder Spulenkern 25' umfasst zusätzlich zu dem Längsschenkel 26' einen Querschenkel 27', welcher jeweils an dem zweiten Ende des Längsschenkels 26' vorgesehen ist, und welcher sich in radialer Richtung nach innen erstreckt, also im Wesentlichen rechtwinklig zum Längsschenkel 26`. Die Spulenkerne 25' haben jeweils die Form eines L, wobei die Querschenkel 27' die kurzen Schenkel des L bilden. Der Rotor 3' ist dann zwischen den Querschenkeln 27` angeordnet. In Fig. 1 und in Fig. 2 ist von dem Rotor 3' nur der magnetisch wirksame Kern 31' des Rotors 3' dargestellt, welcher hier als scheibenförmiger Permanentmagnet ausgestaltet ist. Die Magnetisierung des Permanentmagneten ist durch den Pfeil ohne Bezugszeichen dargestellt.

Die Mehrzahl der Längsschenkel 26`, die sich in axialer Richtung A' erstrecken und an die Säulen eines Tempels erinnern, hat dem Tempelmotor seinen Namen gegeben.

Bei der in Fig. 1 und Fig. 2 dargestellten Ausgestaltung des Tempelmotors 1' als lagerloser Motor sind die Spulenkerne 25' - hier beispielsweise sechs Spulenkerne 25' - mit den Längsschenkeln 26' und den Querschenkeln 27` kreisförmig und äquidistant um den Rotor 3' herum angeordnet (Innenläufer). Die ersten Enden der Längsschenkel 26` sind in radialer Richtung durch einen Rückschluss 22' verbunden, welcher der magnetischen Flussführung dient. Der permanentmagnetische, scheibenförmige Rotor 3' ist zwischen den radial innenliegenden Enden der Querschenkel 27' angeordnet und rotiert im Betriebszustand um die axiale Richtung A', wobei der Rotor 3' berührungslos magnetisch angetrieben und berührungslos magnetisch bezüglich des Stators 2' gelagert ist, und wobei die radiale Position des Rotors 3' so geregelt wird, dass er sich in einer zentrierten Position zwischen den Querschenkeln 27` befindet.

Um die für den magnetischen Antrieb und die magnetische Lagerung des Rotors 3' notwendigen elektromagnetischen Drehfelder zu erzeugen, tragen die Längsschenkel 26` Wicklungen. Die Wicklungen sind beispielsweise so ausgestaltet, dass um jeden Längsschenkel 26' herum eine konzentrierte Wicklung 61' gewickelt ist, das heisst, die Spulenachse jeder konzentrierten Wicklung 61' erstreckt sich jeweils in axialer Richtung A'. Dabei ist es typisch für den Tempelmotor, dass die Spulenachsen der konzentrierten Wicklungen 61' parallel zur Solldrehachse verlaufen und, dass die konzentrierten Wicklungen 61'nicht in der radialen Ebene E' angeordnet sind. Die radiale Ebene E' ist diejenige Ebene senkrecht zur axialen Richtung A', in welcher der Rotor 3' bzw. der magnetisch wirksame Kern 31' des Rotors 3' im Betriebszustand gelagert wird.

Einer der Vorteile der Ausgestaltung als Tempelmotor ist es, dass in der radialen Ebene E' keine Wicklungen oder Wickelköpfe des Stators vorhanden sind. Dies ermöglicht es beispielsweise bei einer Anwendung des Tempelmotors in einer Zentrifugalpumpe, dass der Auslass der Zentrifugalpumpe in der Ebene vorgesehen werden kann, in welcher das Flügelrad des Pumpenrotors rotiert, der Auslass also auf gleicher Höhe bezüglich der axialen Richtung A' liegt wie die Flügel des Pumpenrotors, ohne dass dabei die Wicklungen des Stators stören. Diese zentrale, d.h. mittige Anordnung des Pumpenauslasses ist unter hydrodynamischen Aspekten und im Hinblick speziell auf die passive Lagerung und Stabilisierung des Rotors gegen Verkippungen besonders günstig.

Im Hinblick auf eine möglichst kompakte und leistungsstarke Ausgestaltung des Tempelmotors 1', wie sie beispielsweise auch in Blutpumpen benötigt wird, können verschiedene Massnahmen in Erwägung gezogen werden, deren gleichzeitige Realisierung jedoch nur durch Kompromisse oder Optimierungen möglich ist, weil diese Massnahmen teilweise gegenläufig sind. Im Hinblick auf eine möglichst kompakte Ausgestaltung ist man beispielsweise bemüht, die Längsschenkel 26' auf einem möglichst kleinen Radius anzuordnen. Andererseits ist man bemüht, den magnetisch wirksamen Kern 31' des Rotors 3' mit einem möglichst grossen Durchmesser auszugestalten, weil das auf den Rotor 3' ausübbare Drehmoment - und damit beispielsweise die Leistungsfähigkeit einer Zentrifugalpumpe - im Wesentlichen proportional zum Quadrat des Durchmessers des magnetisch wirksamen Kerns 31' ist. Ein grösserer Durchmesser des magnetisch wirksamen Kerns 31' verlangt dann aber danach, die Längsschenkel 26' auf einem grösseren Radius anzuordnen, damit der Rotor 3' noch zwischen den Querschenkeln 27' der Spulenkerne 25' angeordnet werden kann.

Zudem ist es insbesondere im Hinblick auf die passive magnetische Stabilisierung des Rotors 3' vorteilhaft, wenn das Verhältnis aus dem Durchmesser und der in axialer Richtung A' gemessenen Höhe des magnetisch wirksamen Kerns 31' in einem bestimmten Bereich liegt. Vergrössert man nun den Durchmesser des magnetisch wirksamen Kerns 31', so sollte auch seine Höhe vergrössert werden, damit das Verhältnis aus Durchmesser und Höhe zumindest ungefähr konstant bleibt. Eine grössere Höhe des magnetisch wirksamen Kerns 31' kann aber dazu führen, dass der magnetische Fluss aus dem magnetisch wirksamen Kern 31' des Rotors 3' nicht mehr vollständig in die Querschenkel 27' geführt wird (so wie in Fig. 2 gezeigt), sondern wie in Fig. 2a dargestellt, teilweise aus dem magnetisch wirksamen Kern 31' direkt - also nicht über die Querschenkel 27' - in die Längsschenkel 26' der Spulenkerne 25' geführt wird. Dies führt in der Summe dazu, dass der magnetisch wirksame Kern 31' nicht mehr korrekt in der radialen Ebene E' gelagert ist, sondern darstellungsgemäss nach unten ausgelenkt wird (Fig. 2a). Das hat dann wiederum zur Folge, dass, beispielsweise bei Anwendung des lagerlosen Motors in einer Pumpe, das Flügelrad, welches den Rotor 3' mit dem magnetisch wirksamen Kern 31' umfasst, teilweise in den Stator 2' hineingezogen wird und somit teilweise im Stator 2' des Motors 1' verschwindet, und nicht mehr mit dem Auslass bzw. dem Auslasskanal ausgerichtet ist, sondern relativ zu diesem in axialer Richtung verschoben ist. Zusätzlich wird durch die Auslenkung des Rotors 3' nach unten die maximale Axialkraft der passiven magnetischen Lagerung verringert.

Ausgehend von diesem Stand der Technik ist es daher eine Aufgabe der Erfindung, einen elektromagnetischen Drehantrieb vorzuschlagen, der als Tempelmotor ausgestaltet ist, und der einen magnetisch berührungslos antreibbaren sowie magnetisch berührungslos lagerbaren Rotor umfasst, wobei der elektromagnetische Drehantrieb sehr kompakt ausgestaltet werden kann, ohne dass dafür Zugeständnisse an das auf den Rotor ausübbare Drehmoment oder an die Qualität der magnetischen Lagerung des Rotors vonnöten sind. Zudem ist es eine Aufgabe der Erfindung, eine Zentrifugalpumpe vorzuschlagen, die einen solchen Drehantrieb umfasst. Ferner soll durch die Erfindung eine Pumpeneinheit für eine solche Zentrifugalpumpe vorgeschlagen werden, die insbesondere auch für den Einmalgebrauch ausgestaltet werden kann.

Die diese Aufgabe lösenden Gegenstände der Erfindung sind durch die Merkmale des unabhängigen Patentanspruchs der jeweiligen Kategorie gekennzeichnet.

Erfindungsgemäss wird also ein elektromagnetischer Drehantrieb vorgeschlagen, der als Tempelmotor ausgestaltet ist, mit einem Rotor, der einen ring- oder scheibenförmigen magnetisch wirksamen Kern umfasst, sowie mit einem Stator, welcher als Lager- und Antriebsstator ausgestaltet ist, mit welchem der Rotor im Betriebszustand berührungslos magnetisch um eine Solldrehachse antreibbar ist, die eine axiale Richtung definiert, und mit welchem der Rotor berührungslos magnetisch bezüglich des Stators lagerbar ist, wobei der Rotor in einer zur axialen Richtung senkrechten radialen Ebene aktiv magnetisch gelagert ist, und in axialer Richtung sowie gegen Verkippungen passiv magnetisch stabilisiert ist, wobei der Stator eine Mehrzahl von Spulenkernen aufweist, von denen jeder einen Längsschenkel umfasst, welcher sich in axialer Richtung erstreckt, sowie einen in der radialen Ebene angeordneten Querschenkel, welcher sich von dem Längsschenkel in einer radialen Richtung erstreckt, und durch eine Stirnfläche begrenzt wird, welche dem magnetisch wirksamen Kern des Rotors zugewandt ist, und wobei an jedem Längsschenkel mindestens eine konzentrierte Wicklung angeordnet ist, welche den jeweiligen Längsschenkel umgibt. Jeder Längsschenkel umfasst einen ersten Abschnitt und einen zweiten Abschnitt, welche bezüglich der axialen Richtung benachbart zueinander angeordnet sind, wobei der Querschenkel an dem zweiten Abschnitt angeordnet ist, wobei die Stirnfläche des Querschenkels von dem ersten Abschnitt des zugehörigen Längsschenkels einen ersten Abstand in radialer Richtung aufweist, und von dem zweiten Abschnitt einen zweiten Abstand in radialer Richtung, und wobei der zweite Abstand grösser ist als der erste Abstand.

Durch diese Ausgestaltung können die Spulenkerne an ihren den Querschenkeln abgewandten Enden auf einem Kreis mit kleinerem Radius angeordnet werden, was eine sehr kompakte Ausgestaltung des Stators ermöglicht. Gleichzeitig weisen die Längsschenkel in ihrem zweiten Abschnitt einen grösseren diametralen Abstand voneinander auf, sodass der Durchmesser des magnetisch wirksamen Kerns vergrössert werden kann, ohne dass die Gefahr besteht, dass der magnetische Fluss aus dem Rotor direkt in die Längsschenkel der Spulenkerne übertritt. Insbesondere die Vergrösserung des Durchmessers des magnetisch wirksamen Kerns des Rotors wirkt sich vorteilhaft auf die Leistungsfähigkeit des elektromagnetischen Drehantriebs aus, weil hierdurch das auf den Rotor einwirkende Drehmoment vergrössert werden kann.

In einer bevorzugten Ausgestaltung sind für jeden Längsschenkel der erste Abschnitt und der zweite Abschnitt parallel zueinander angeordnet, und durch einen Übergangsbereich miteinander verbunden, in welchem sich der radiale Abstand von der zugehörigen Stirnfläche von dem ersten Abstand zu dem zweiten Abstand ändert. Dies kann beispielsweise durch eine gekröpfte Ausgestaltung der Spulenkerne realisiert werden.

Ferner ist es vorteilhaft, wenn der magnetisch wirksame Kern des Rotors eine Rotorhöhe aufweist, welche die maximale Erstreckung des magnetisch wirksamen Kerns in axialer Richtung ist, wobei die Rotorhöhe grösser ist als eine Statorpolhöhe, welche durch die maximale Erstreckung der Stirnflächen der Querschenkel in axialer Richtung festgelegt ist.

Besonders bevorzugt ist der magnetisch wirksame Kern des Rotors permanentmagnetisch ausgestaltet, das heisst er umfasst mindestens einen Permanentmagneten oder er besteht aus einem permanentmagnetischen Material. Ferner ist es bevorzugt, dass jede Stirnfläche der Querschenkel in der axialen Richtung die gleiche Erstreckung hat, sodass für jede Stirnfläche ihre jeweilige Erstreckung in axialer Richtung gleich der Statorpolhöhe ist.

Dadurch, dass der magnetisch wirksame Kern des Rotor die Rotorhöhe aufweist, die grösser ist als die Statorpolhöhe, resultiert eine Konzentration des magnetischen Flusses im Luftspalt zwischen den Stirnflächen und dem magnetisch wirksamen Kern des Rotors, das heisst die magnetische Flussdichte nimmt im Luftspalt zu. Da die axiale Steifigkeit der magnetischen Stabilisierung des Rotors zumindest näherungsweise quadratisch mit der magnetischen Flussdichte im Luftspalt zunimmt, nimmt die axiale Steifigkeit überproportional mit der Rotorhöhe zu. Somit resultiert daraus, dass die Rotorhöhe grösser ist als die Statorpolhöhe eine Vergrösserung der axialen Steifigkeit der magnetischen Lagerung bzw. Stabilisierung des Rotors.

Um die Kippsteifigkeit des Rotors, also seinen Widerstand gegen Verkippungen relativ zu der radialen Ebene, die senkrecht zur axialen Richtung liegt, nicht zu stark zu reduzieren, ist es bevorzugt, dass das Verhältnis aus dem Aussendurchmesser des magnetisch wirksamen Kerns des Rotors und der Rotorhöhe den Wert von 2.8 nicht unterschreitet.

Ferner ist es eine bevorzugte Massnahme, dass der zweite Abstand grösser ist als die Hälfte einer Statorpolhöhe, welche durch die maximale Erstreckung der Stirnflächen der Querschenkel in axialer Richtung festgelegt ist. Hierdurch lässt es sich besonders effizient vermeiden, dass der magnetische Fluss aus dem magnetisch wirksamen Kern des Rotors direkt in die Längsschenkel der Spulenkerne übertritt und umgekehrt.

Bei einer bevorzugten Ausführungsform umfasst der magnetisch wirksame Kern einen zentralen Bereich, welcher bezüglich der axialen Richtung zwischen einem ersten Randbereich und einem zweiten Randbereich angeordnet ist, und welcher einen Rotordurchmesser aufweist, wobei der erste Randbereich eine erste axiale Begrenzungsfläche des magnetisch wirksamen Kerns bildet, die einen ersten Randdurchmesser aufweist, wobei der zweite Randbereich eine zweite axiale Begrenzungsfläche des magnetisch wirksamen Kerns bildet, die einen zweiten Randdurchmesser aufweist, und wobei jeder Randdurchmesser kleiner ist als der Rotordurchmesser.

Bei dieser Ausführungsform kann somit der radial aussenliegende Bereich des magnetischen Kerns des Rotors mit einer geringeren Höhe - gemessen in der axialen Richtung - ausgestaltet werden, als der Bereich zwischen der ersten und der zweiten axialen Begrenzungsfläche. Dies hat den Vorteil, dass ebenfalls eine Vergrösserung der axialen Steifigkeit der magnetischen Stabilisierung resultiert, aber eine deutlich geringere Abnahme der Kippsteifigkeit.

Vorzugsweise weist der zentrale Bereich eine zentrale Höhe auf, welches die Erstreckung des zentralen Bereichs in axialer Richtung ist, wobei die zentrale Höhe gleich gross ist wie die Statorpolhöhe. Dies hat den Vorteil, dass der radial aussenliegende Bereich des magnetisch wirksamen Kerns des Rotors, also derjenige Bereich, welcher den Stirnflächen der Querschenkel direkt gegenüber liegt, in der axialen Richtung die gleiche Erstreckung hat wie die Stirnflächen der Querschenkel, sodass im Luftspalt eine sehr hohe magnetische Flussdichte resultiert, was auch im Hinblick auf das Drehmoment vorteilhaft ist, welches die Rotation des Rotors antreibt.

Eine weitere bevorzugte Massnahme ist es, dass der magnetisch wirksame Kern eine Aussenfläche aufweist, die weder zwischen dem zentralen Bereich und der ersten axialen Begrenzungsfläche noch zwischen dem zentralen Bereich und der zweiten axialen Begrenzungsfläche parallel zur axialen Richtung verläuft. Hierdurch lässt es sich realisieren, dass die Feldlinien des magnetischen Flusses zwischen dem zentralen Bereich und den beiden axialen Begrenzungsflächen nicht senkrecht zur axialen Richtung aus dem magnetisch wirksamen Kern des Rotors austreten, sondern unter einem Winkel der kleiner ist als 90°. Hierdurch treten die Feldlinien bezüglich der radialen Richtung weiter aussen in die Querschenkel der Spulenkerne ein, wodurch sich die magnetische Lagerung bzw. Stabilisierung des Rotors verbessert.

Besonders bevorzugt ist es, dass mindestens einer des ersten und des zweiten Randbereichs in Form eines Kegelstumpfs oder in Form einer Kugelscheibe oder in Form einer Paraboloidscheibe ausgestaltet ist. Durch solche Ausgestaltungen lässt es sich insbesondere realisieren, dass die Aussenfläche, welche den magnetisch wirksamen Kern des Rotors begrenzt, sowohl zwischen dem zentralen Bereich und der ersten axialen Begrenzungsfläche als auch zwischen dem zentralen Bereich und der zweiten axialen Begrenzungsfläche nicht parallel zur axialen Richtung verläuft, sodass hier die Feldlinien des magnetischen Flusses unter einem von 90° verschiedenen Winkel aus dem magnetisch wirksamen Kern des Rotors austreten bzw. in diesen eintreten.

Der erste und der zweite Randbereich können unterschiedlich ausgestaltet sein. So kann beispielsweise der erste Randbereich kegelstumpfförmig ausgestaltet sein und der zweite Randbereich in Form einer Kugelscheibe. Natürlich ist es auch möglich, dass der erste Randbereich und der zweite Randbereich gleichartig ausgestaltet sind.

Gemäss einem zweiten bevorzugten Ausführungsbeispiel sind an jedem Längsschenkel zwei konzentrierte Wicklungen vorgesehen, von denen jede den jeweiligen Längsschenkel umgibt, und die bezüglich der axialen Richtung benachbart zueinander angeordnet sind. Hierdurch ist es möglich, eine der konzentrierten Wicklungen als Antriebsspule zu verwenden und die andere der beiden konzentrierten Wicklungen als Steuerspule, wobei dann die Überlagerung der von allen Antriebsspulen und von allen Steuerspulen generierten elektromagnetischen Felder den Antrieb und die berührungslose magnetische Lagerung des Rotors bewirken.

Die auf dem gleichen Längsschenkel axial zueinander benachbart angeordneten beiden konzentrierten Wicklungen sollen sich nicht berühren. Dies kann vorteilhafter Weise so realisiert werden, dass bezüglich der axialen Richtung zwischen diesen beiden konzentrierten Wicklungen eine Kontrolleinrichtung oder ein Teil einer Kontrolleinrichtung zur Ansteuerung und zur Regelung des elektromagnetischen Drehantriebs angeordnet ist. Diese Kontrolleinrichtung kann beispielsweise auch Leistungselektronik für die konzentrierten Wicklungen umfassen. Vorzugsweise ist die zwischen den beiden konzentrierten Wicklungen angeordnete Kontrolleinrichtung als Platine bzw. PCB (printed circuit board) ausgestaltet.

Durch die Erfindung wird ferner eine Zentrifugalpumpe zum Fördern eines Fluids vorgeschlagen, die dadurch gekennzeichnet, dass die Zentrifugalpumpe einen elektromagnetischen Drehantrieb umfasst, der erfindungsgemäss ausgestaltet ist, wobei der Rotor des elektromagnetischen Drehantriebs als Rotor der Zentrifugalpumpe ausgestaltet ist.

Vorzugsweise umfasst die Zentrifugalpumpe eine Pumpeneinheit mit einem Pumpengehäuse, das einen Einlass und einen Auslass für das zu fördernde Fluid umfasst, wobei der Rotor im Pumpengehäuse angeordnet ist, und eine Mehrzahl von Flügeln zum Fördern des Fluids umfasst, wobei die Pumpeneinheit derart ausgestaltet ist, dass die Pumpeneinheit so in den Stator einsetzbar ist, dass der magnetisch wirksame Kern des Rotors von den Stirnflächen der Querschenkel umgeben wird.

Ferner wird durch die Erfindung eine Pumpeneinheit für eine Zentrifugalpumpe vorgeschlagen, welche dadurch gekennzeichnet ist, dass die Pumpeneinheit für eine Zentrifugalpumpe ausgestaltet ist, welche gemäss der Erfindung ausgestaltet ist.

Gemäss einer bevorzugten Ausgestaltung umfasst das Pumpengehäuse ein Basisteil und einen Deckel, welche dichtend miteinander verbunden sind, wobei der Auslass des Pumpengehäuses vollständig im Basisteil angeordnet ist.

Auch ist es eine bevorzugte Massnahme, dass der Rotor eine zentrale Bohrung aufweist, welche sich in axialer Richtung vollständig durch den Rotor hindurch erstreckt, sodass der durch die Flügel generierte Axialschub zumindest teilweise kompensiert wird.

Vorzugsweise ist die Pumpeneinheit zum lösbaren Verbinden mit dem Stator der erfindungsgemässen Zentrifugalpumpe ausgestaltet.

Gemäss einer bevorzugten Ausgestaltung ist die Pumpeneinheit als Einmalvorrichtung für den Einmalgebrauch ausgestaltet.

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher erläutert. In der teilweise schematischen Zeichnung zeigen:
- Fig. 1:: eine perspektivische Darstellung eines Tempelmotors gemäss Stand der Technik,
- Fig. 2:: eine schematische Darstellung des Tempelmotors aus Fig. 1 in einem Schnitt in axialer Richtung,
- Fig. 2a:: wie Fig. 2, wobei jedoch der Rotor bezüglich der axialen Richtung ausgelenkt ist,
- Fig. 3: ein erstes Ausführungsbeispiel eines erfindungsgemässen elektromagnetischen Drehantriebs in einem Schnitt in axialer Richtung,
- Fig. 4:: eine Aufsicht auf das erste Ausführungsbeispiel aus der axialen Richtung,
- Fig. 5:: eine Ansicht eines einzelnen Spulenkerns,
- Fig. 6:: eine perspektivische Ansicht des Spulenkerns,
- Fig. 7:: eine perspektivische Ansicht eines Blechelements eines Spulenkerns,
- Fig. 8:: wie Fig. 3, jedoch mit einer ersten Variante für den magnetisch wirksamen Kern des Rotors
- Fig. 9:: den magnetisch wirksamen Kern des Rotors aus Fig. 8,
- Fig. 10:: eine zweite Variante für den magnetisch wirksamen Kern des Rotors,
- Fig. 11:: eine dritte Variante für den magnetisch wirksamen Kern des Rotors in einem Schnitt in axialer Richtung.
- Fig. 12:: eine vierte Variante für den magnetisch wirksamen Kern des Rotors,
- Fig. 13:: eine fünfte Variante für den magnetisch wirksamen Kern des Rotors,
- Fig. 14:: eine perspektivische Darstellung eines zweiten Ausführungsbeispiels eines erfindungsgemässen elektromagnetischen Drehantriebs,
- Fig. 15:: das zweiten Ausführungsbeispiels des erfindungsgemässen elektromagnetischen Drehantriebs in einem Schnitt in axialer Richtung,
- Fig. 16:: ein Ausführungsbeispiel einer erfindungsgemässen Zentrifugalpumpe in einem Schnitt in axialer Richtung,
- Fig. 17:: die Pumpeneinheit der Zentrifugalpumpe aus Fig. 12 in einem axialen Schnitt entlang der Schnittlinie XVII-XVII in Fig. 18, und
- Fig. 18:: eine Aufsicht auf die Pumpeneinheit aus Fig. 17 aus der axialen Richtung.

Wie bereits erwähnt und erläutert, ist in Fig. 1 und in Fig. 2 ein als Tempelmotor ausgestalteter elektromagnetischer Drehantrieb dargestellt, der aus dem Stand der Technik bekannt ist.

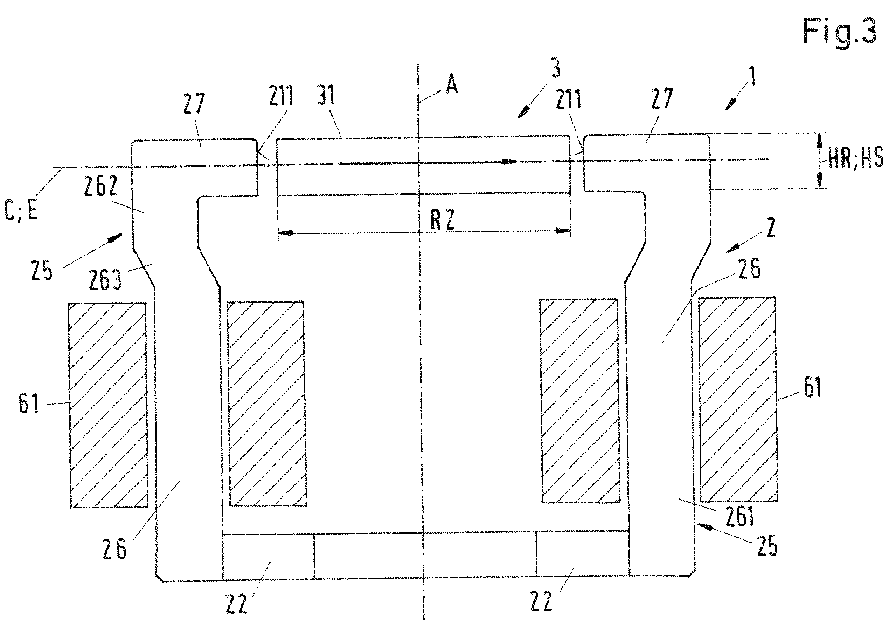

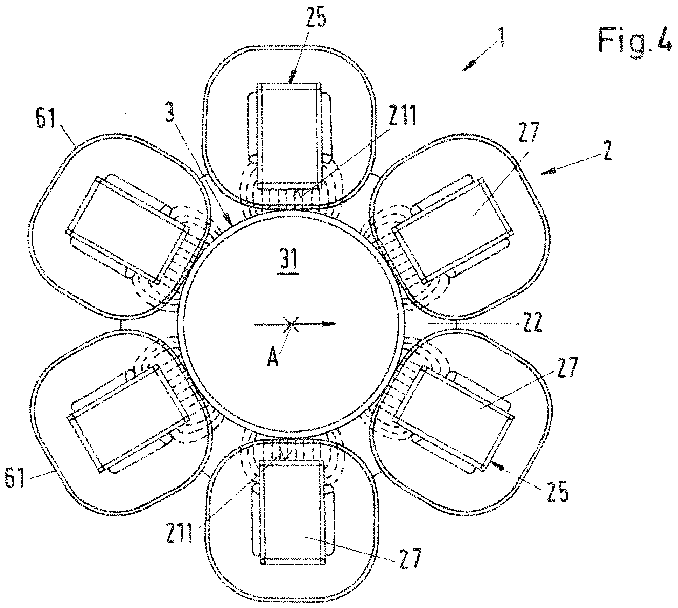

Fig. 3 zeigt einen Schnitt in axialer Richtung durch ein erstes Ausführungsbeispiel eines erfindungsgemässen Drehantriebs, der gesamthaft mit dem Bezugszeichen 1 bezeichnet ist. Zum besseren Verständnis zeigt Fig. 4 noch eine Aufsicht auf das erste Ausführungsbeispiel aus der axialen Richtung.

Der elektromagnetische Drehantrieb 1 ist als Tempelmotor ausgestaltet und umfasst einen Stator 2, welcher eine Mehrzahl von Spulenkernen 25 - hier sechs Spulenkerne 25 - aufweist, von denen jeder einen Längsschenkel 26 umfasst, welcher sich in axialer Richtung A erstreckt, sowie einen senkrecht zum Längsschenkel 26 angeordneten Querschenkel 27, welcher sich in einer radialen Richtung erstreckt, und durch eine Stirnfläche 211 begrenzt wird. Die Spulenkerne 25 sind äquidistant auf einer Kreislinie angeordnet, sodass die Stirnflächen 211 einen Rotor 3 des elektromagnetischen Drehantriebs 1 umgeben. An jedem Längsschenkel 26 ist eine konzentrierte Wicklung 61 angeordnet, welche den jeweiligen Längsschenkel 26 umgibt.

Der Rotor 3 ist bezüglich des Stators 2 berührungslos magnetisch gelagert. Ferner ist der Rotor 3 mittels des Stators 2 berührungslos magnetisch zur Rotation um eine Solldrehachse antreibbar. Die Solldrehachse bezeichnet dabei diejenige Achse, um welche sich der Rotor 3 im Betriebszustand dreht, wenn sich der Rotor 3 bezüglich des Stators 2 in einer zentrierten und unverkippten Lage befindet, so wie dies in Fig. 3 dargestellt ist. Diese Solldrehachse definiert eine axiale Richtung A. Üblicherweise stimmt die die axiale Richtung A festlegende Solldrehachse mit der Mittelachse des Stators 2 überein.

Im Folgenden wird mit einer radialen Richtung eine Richtung bezeichnet, welche senkrecht auf der axialen Richtung A steht.

Der Rotor 3 umfasst einen magnetisch wirksamen Kern 31, welcher ring- oder scheibenförmig ausgestaltet ist. Der magnetisch wirksame Kern 31 ist gemäss der Darstellung in Fig. 3 als Scheibe ausgestaltet und definiert eine magnetische Mittelebene C. Mit der magnetischen Mittelebene C des magnetisch wirksamen Kerns 31 des Rotors 3 wird diejenige Ebene senkrecht zur axialen Richtung A bezeichnet, in welcher der magnetisch wirksame Kern 31 des Rotors 3 im Betriebszustand gelagert wird, wenn der Rotor 3 nicht verkippt und in axialer Richtung A nicht ausgelenkt ist. In der Regel ist bei einem scheibenförmigen oder ringförmigen magnetisch wirksamen Kern 31 die magnetische Mittelebene C die geometrische Mittelebene des magnetisch wirksamen Kerns 31 des Rotors 3, die senkrecht zur axialen Richtung A liegt. Diejenige Ebene, in welcher der magnetisch wirksame Kern 31 des Rotors 3 im Betriebszustand zwischen den Stirnflächen 211 im Stator 2 gelagert ist, wird auch als radiale Ebene E bezeichnet. Die radiale Ebene definiert die x-y-Ebene eines kartesischen Koordinatensystems, dessen z-Achse in axialer Richtung A verläuft. Ist der magnetisch wirksame Kern 31 des Rotors 3 nicht verkippt und bezüglich der axialen Richtung (A) nicht ausgelenkt, so stimmt die radiale Ebene E mit der magnetischen Mittelebene C überein.

Mit der radialen Position des magnetisch wirksamen Kerns 31 bzw. des Rotors 3 wird die Lage des Rotors 3 in der radialen Ebene E bezeichnet.

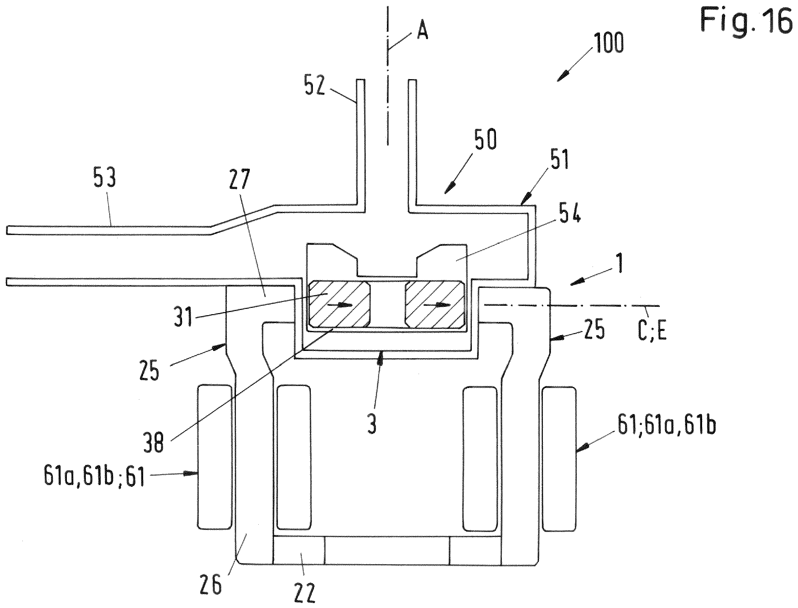

Da es für das Verständnis der Erfindung ausreichend ist, ist in der Zeichnung, z. B. in den Fig. 3, 4, und 8-15, von dem Rotor 3 jeweils nur der magnetisch wirksame Kern 31 dargestellt. Es versteht sich, dass der Rotor 3 natürlich auch noch weitere Komponenten umfassen kann wie beispielsweise Ummantelungen oder Kapselungen, die vorzugsweise aus einem Kunststoff hergestellt sind, oder aus einem Metall oder aus einer Metalllegierung oder aus einer Keramik bzw. einem keramischen Werkstoff. Ferner kann der Rotor 3 auch Flügel zum Mischen, Rühren oder Pumpen von Fluiden (siehe z. B. Fig. 16) oder sonstige Komponenten umfassen.

Der Rotor 3 und insbesondere der magnetisch wirksame Kern 31 des Rotors 3 ist von den radial aussenliegend angeordneten Querschenkeln 27 der Spulenkerne 25 des Stators 2 umgeben. Die Querschenkel 27 bilden somit eine Mehrzahl von ausgeprägten Statorpolenhier sechs Statorpole. Die Längsschenkel 26 der Spulenkerne 25 erstrecken sich jeweils von einem ersten Ende, welches das darstellungsgemäss untere Ende ist, in axialer Richtung A bis zu einem zweiten Ende, welches das darstellungsgemäss obere Ende ist. Die Querschenkel 27 sind an den oberen Enden der Längsschenkel 26 angeordnet. Jeder Querschenkel 27 erstreckt sich in der radialen Richtung auf den Rotor 3 zu.

Wenn sich der magnetisch wirksame Kern 31 des Rotors 3 während des Betriebs in seiner Soll-Position befindet, so ist der magnetisch wirksame Kern 31 zwischen den Stirnflächen 211 der Querschenkel 27 zentriert, sodass die Querschenkel 27 in der magnetischen Mittelebene C bzw. in der radialen Ebene E (diese zwei Ebenen sind in diesem Fall gleich) angeordnet sind. Die konzentrierten Wicklungen 61 sind darstellungsgemäss unterhalb der radialen Ebene E angeordnet und so ausgerichtet, dass ihre Spulenachsen in axialer Richtung A verlaufen.

Alle ersten Enden der Längsschenkel 26 - also die darstellungsgemäss unteren Enden - sind durch einen Rückschluss 22 miteinander verbunden. Der Rückschluss 22 ist vorzugsweise ringförmig ausgestaltet. Dabei sind solche Ausgestaltungen möglich (siehe Fig. 3), bei welchen sich der Rückschluss 22 radial innenliegend entlang aller ersten Enden der Längsschenkel 26 erstreckt. Es ist aber auch möglich, dass der Rückschluss 22 entlang seines Umfangs mehrere Ausnehmungen aufweist, von denen jede eines der ersten Enden aufnimmt. In anderen Ausführungsformen kann der Rückschluss auch eine Mehrzahl von Ringsegmenten umfassen, von denen jedes jeweils zwischen zwei in Umfangsrichtung benachbarten Spulenkernen 25 im Bereich der ersten Enden angeordnet ist.

Um die für den magnetischen Antrieb und die magnetische Lagerung des Rotors 3 notwendigen elektromagnetischen Drehfelder zu erzeugen, tragen die Längsschenkel 26 der Spulenkerne 25 die als konzentrierte Wicklungen 61 ausgestalteten Wicklungen, wobei bei dem ersten Ausführungsbeispiel um jeden Längsschenkel 26 herum jeweils genau eine konzentrierte Wicklung 61 angeordnet ist. Mit diesen konzentrierten Wicklungen 61 werden im Betriebszustand diejenigen elektromagnetischen Drehfelder erzeugt, mit welchen ein Drehmoment auf den Rotor 3 bewirkt wird, und mit welchen eine beliebig einstellbare Querkraft in radialer Richtung auf den Rotor 3 ausübbar ist, sodass die radiale Position des Rotors 3, also seine Position in der zur axialen Richtung A senkrechten radialen Ebene E, aktiv steuerbar bzw. regelbar ist.

Mit dem "magnetisch wirksamen Kern 31" des Rotors 3 ist derjenige Bereich des Rotors 3 gemeint, welcher für die Drehmomentbildung sowie für die Erzeugung der magnetischen Lagerkräfte magnetisch mit dem Stator 2 zusammenwirkt.

Wie bereits erwähnt, ist der magnetisch wirksame Kern 31 scheibenförmig ausgestaltet. Ferner ist der magnetisch wirksame Kern 31 permanentmagnetisch ausgestaltet. Dazu kann der magnetisch wirksame Kern 31 mindestens einen Permanentmagneten, aber auch mehrere Permanentmagnete umfassen oder - wie im hier beschriebenen Ausführungsbeispiel - vollständig aus einem permanentmagnetischen Material bestehen, sodass der magnetisch wirksame Kern 31 der Permanentmagnet ist. Die Magnetisierung des magnetisch wirksamen Kerns des Rotors 3 ist in Fig. 3 und in Fig. 4 jeweils durch den Pfeil ohne Bezugszeichen im magnetisch wirksamen Kern 31 dargestellt. Der magnetisch wirksame Kern 31 ist also in radialer Richtung magnetisiert.

Als Permanentmagnete bezeichnet man üblicherweise solche ferromagnetischen oder ferrimagnetischen Werkstoffe, die hartmagnetisch sind, also eine hohe Koerzitivfeldstärke aufweisen. Die Koerzitivfeldstärke ist diejenige magnetische Feldstärke, die man benötigt, um einen Stoff zu entmagnetisieren. Im Rahmen dieser Anmeldung wird unter einem Permanentmagneten ein Werkstoff bzw. ein Material verstanden, der eine Koerzitivfeldstärke, genauer gesagt eine Koerzitivfeldstärke der magnetischen Polarisation aufweist, die mehr als 10`000 A/m beträgt.

Sowohl der ringförmige Rückschluss 22 als auch die Spulenkerne 25 des Stators 2 sind jeweils aus einem weichmagnetischen Material gefertigt, weil sie als Flussleitelemente zur Führung des magnetischen Flusses dienen.

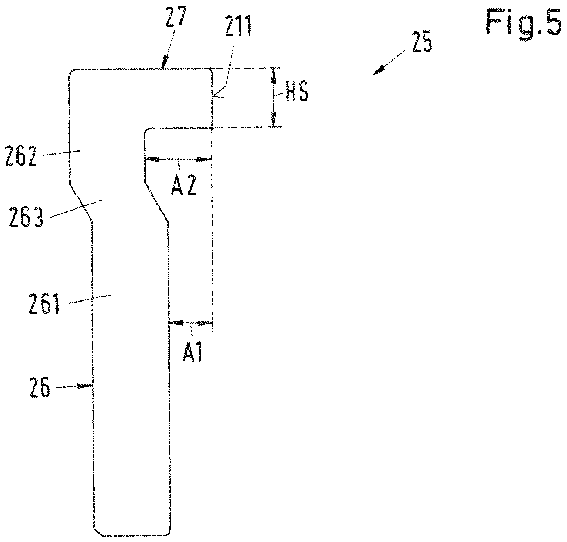

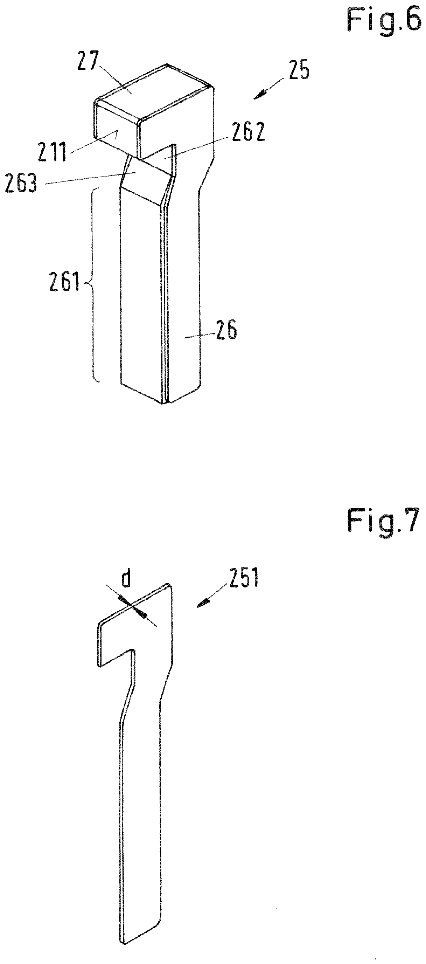

Fig. 5 zeigt zum besseren Verständnis eine Ansicht eines einzelnen Spulenkerns 25 und Fig. 6 eine perspektivische Ansicht des Spulenkerns 25.

Geeignete weichmagnetische Materialien für die Spulenkerne 25 und den Rückschluss 22 sind beispielsweise ferromagnetische oder ferrimagnetische Materialien, also insbesondere Eisen, Nickel-Eisen, Kobalt-Eisen Silizium-Eisen oder Mu-Metall. Hierbei ist für den Stator 2 eine Ausgestaltung als Statorblechpaket bevorzugt, bei welcher die Spulenkerne 25 und der Rückschluss 22, geblecht ausgestaltet sind, das heisst sie bestehen aus mehreren dünnen Blechelementen, die gestapelt sind.

Für einen Spulenkern 25 zeigt Fig. 7 eine perspektivische Ansicht eines solchen Blechelements 251. Eine Vielzahl solcher Blechelemente 251 wird dann gestapelt, um einen Spulenkern 25 herzustellen. Ein einzelnes Blechelement 251 eines ferromagnetischen Spulenkerns 25 hat typischerweise eine Blechdicke d von 0.2 mm bis 0.5 mm. Üblicherweise sind die Wirbelstromverluste umso geringer je dünner die Blechelements 251 sind, d.h. je kleiner die Blechdicke d ist.

Ferner ist es möglich, dass die Spulenkerne 25 und der Rückschluss 22 aus gepressten und anschliessend gesinterten Körnern der genannten Materialien bestehen. Die metallischen Körner sind dabei vorzugsweise in eine Kunststoffmatrix eingebettet, sodass sie zumindest teilweise gegeneinander isoliert sind, wodurch sich Wirbelstromverluste minimieren lassen. Es sind also für den Stator auch weichmagnetische Verbundwerkstoffe geeignet, welche aus elektrisch isolierten und zusammengepressten Metallpartikeln bestehen. Insbesondere können diese weichmagnetischen Verbundwerkstoffe, die auch als SMC (Soft Magnetic Composites) bezeichnet werden, aus Eisenpulverpartikeln bestehen, die mit einer elektrisch isolierenden Schicht beschichtet sind. Diese SMC werden dann mittels pulvermetallurgischer Verfahren zu der gewünschten Ausgestaltung geformt.

Während des Betriebs des elektromagnetischen Drehantriebs 1 wirkt der magnetisch wirksame Kern 31 des Rotors 3 mit dem Stator 2 nach dem eingangs beschriebenen Prinzip des lagerlosen Motors zusammen, bei welchem der Rotor 3 berührungslos magnetisch antreibbar und berührungslos magnetisch bezüglich des Stators 2 lagerbar ist. Dazu ist der Stator 2 als Lager- und Antriebsstator ausgestaltet, mit welchem der Rotor 3 im Betriebszustand berührungslos magnetisch um die Solldrehachse antreibbar - also in Rotation versetzbar- und bezüglich des Stators 2 berührungslos magnetisch lagerbar ist. Dabei sind drei Freiheitsgrade des Rotors 3, nämlich seine Position in der radialen Ebene E und seine Rotation, aktiv regelbar. Bezüglich seiner axialen Auslenkung aus der radialen Ebene E in axialer Richtung A ist der magnetisch wirksame Kern 31 des Rotors 3 passiv magnetisch, das heisst nicht ansteuerbar, durch Reluktanzkräfte stabilisiert. Auch bezüglich der verbleibenden zwei Freiheitsgrade, nämlich Verkippungen bezüglich der zur Solldrehachse senkrechten radialen Ebene E ist der magnetisch wirksame Kern 31 des Rotors 3 ebenfalls passiv magnetisch stabilisiert. Der Rotor 3 ist also durch das Zusammenwirken des magnetisch wirksamen Kerns 31 mit den Spulenkernen 25 in axialer Richtung A sowie gegen Verkippungen (insgesamt drei Freiheitsgrade) passiv magnetisch gelagert oder passiv magnetisch stabilisiert und in der radialen Ebene (zwei Freiheitsgrade) aktiv magnetisch gelagert.

Wie dies allgemein üblich ist bezeichnet auch im Rahmen dieser Anmeldung eine aktive magnetische Lagerung eine solche, die aktiv steuer- bzw. regelbar ist, beispielsweise über die mit den konzentrierten Wicklungen 61 generierten elektromagnetischen Drehfelder. Eine passive magnetische Lagerung oder eine passive magnetische Stabilisierung bezeichnet eine solche, die nicht ansteuerbar bzw. regelbar ist. Die passive magnetische Lagerung oder Stabilisierung basiert beispielsweise auf Reluktanzkräften, welche den Rotor 3 bei einer Auslenkung aus seiner Soll-Position, also z. B. bei einer Verschiebung oder Auslenkung in axialer Richtung A oder bei einer Verkippung, wieder in seine Soll-Position bringen.

Mit einer Radiallagerung oder einer radialen Lagerung wird eine Lagerung des Rotors 3 bezeichnet, mit welcher die radiale Position des Rotors 3 stabilisiert werden kann, also eine Lagerung, welche den Rotor 3 in der radialen Ebene E und damit bezüglich seiner radialen Position lagert.

Mit einer Axiallagerung oder einer axialen Lagerung bzw. mit einer Axialstabilisierung oder einer axialen Stabilisierung wird eine Lagerung bzw. eine Stabilisierung des Rotors 3 bezeichnet, mit welcher zum einen die Position des Rotors 3 bezüglich der axialen Richtung A stabilisiert wird, und mit welcher zum anderen der Rotor 3 gegen Verkippungen stabilisiert ist. Solche Verkippungen stellen zwei Freiheitsgrade dar und bezeichnen Auslenkungen, bei denen die momentane Drehachse des Rotors 3 nicht mehr genau in die axiale Richtung A zeigt, sondern einen von Null verschiedenen Winkel mit der Solldrehachse einschliesst. Bei einer Verkippung liegt also die magnetische Mittelebene C nicht mehr in oder parallel zur radialen Ebene E, sondern die magnetische Mittelebene C schliesst einen von Null verschiedenen Winkel mit der radialen Ebene E ein.

Beim lagerlosen Motor werden im Unterschied zu klassischen Magnetlagern die magnetische Lagerung und der Antrieb des Motors über elektromagnetische Drehfelder realisiert. Typischerweise wird in dem lagerlosen Motor die magnetische Antriebs- und Lagerfunktion durch die Überlagerung zweier magnetischer Drehfelder generiert, die üblicherweise als Antriebs- und Steuerfeld bezeichnet werden. Diese beiden mit den Wicklungen des Stators 2 erzeugten Drehfelder haben in der Regel eine Polpaarzahl, die sich um eins unterscheidet. Hat also beispielsweise das Antriebsfeld die Polpaarzahl p, so hat das Steuerfeld die Polpaarzahl p+1 oder p-1. Dabei werden mit dem Antriebsfeld auf den magnetisch wirksamen Kern 31 in der radialen Ebene wirkende Tangentialkräfte erzeugt, die ein Drehmoment bewirken, was die Rotation um die axiale Richtung A bewirkt. Durch die Überlagerung des Antriebsfelds und des Steuerfelds lässt sich zusätzlich eine beliebig einstellbare Querkraft auf den magnetisch wirksame Kern 31 in der radialen Ebene erzeugen, mit welcher die Position des magnetisch wirksamen Kerns 31 in der radialen Ebene regelbar ist. Es ist also nicht möglich, den elektromagnetischen Fluss, der von den konzentrierten Wicklungen 61 generiert wird, aufzuteilen in einen (elektro-) magnetischen Fluss, der nur für den Antrieb der Rotation sorgt und einen (elektro-) magnetischen Fluss, der nur die magnetische Lagerung realisiert.

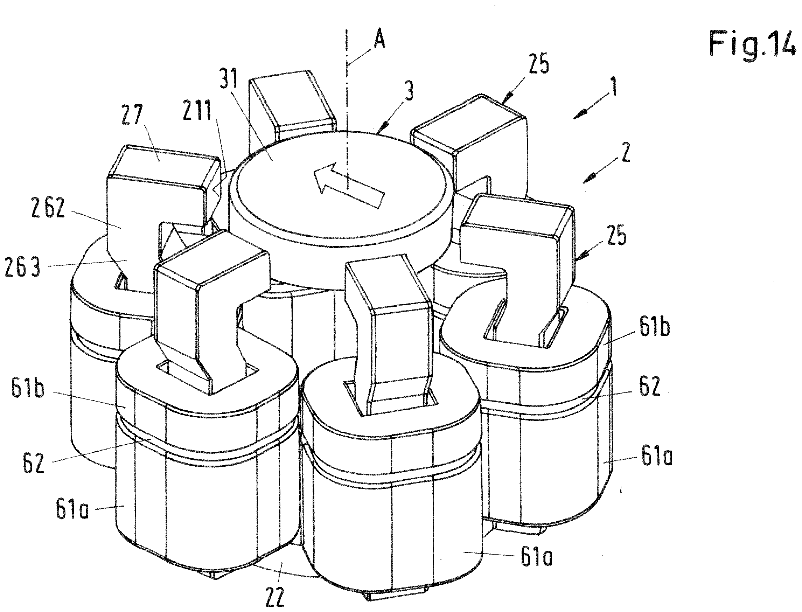

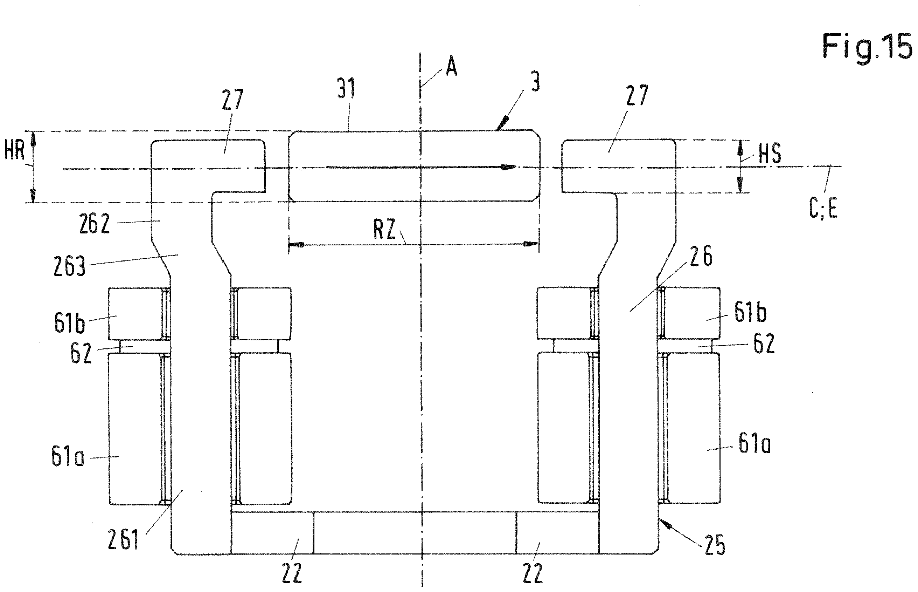

Zur Generierung des Antriebs- und des Steuerfelds ist es einerseits möglich, zwei unterschiedliche Wicklungssysteme zu verwenden, nämlich eines zur Erzeugung des Antriebsfelds und eines zur Erzeugung des Steuerfelds. Die Spulen zur Erzeugung des Antriebsfelds werden dann üblicherweise als Antriebsspulen bezeichnet und die Spulen zur Erzeugung des Steuerfelds als Steuerspulen (siehe hierzu das in Fig. 14 und in Fig. 15 dargestellte zweite Ausführungsbeispiel des erfindungsgemässen Drehantriebs 1). Der Strom, der in diese Spulen eingeprägt wird, wird dann als Antriebsstrom bzw. als Steuerstrom bezeichnet. Andererseits ist es aber auch möglich, die Antriebs- und Lagerfunktion mit nur einem einzigen Wicklungssystem - wie in dem hier beschriebenen ersten Ausführungsbeispiel - zu generieren, sodass es also keine Unterscheidung zwischen Antriebs- und Steuerspulen gibt. Dies kann so realisiert werden, dass die von einer Kontrolleinrichtung jeweils ermittelten Werte für den Antriebs- und den Steuerstrom rechnerisch -also z. B. mit Hilfe von Software - addiert bzw. überlagert werden und der sich daraus ergebende Gesamtstrom in die jeweilige konzentrierte Wicklung 61 eingeprägt wird. In diesem Fall ist es natürlich nicht mehr möglich, zwischen Steuer- und Antriebsspulen zu unterscheiden. In dem hier beschriebenen ersten Ausführungsbeispiel ist die letztgenannte Variante realisiert, das heisst, im Stator 2 gibt es keine Unterscheidung zwischen Antriebs- und Steuerspulen, sondern es gibt jeweils nur ein Wicklungssystem, in dessen sechs konzentrierten Wicklungen 61 die rechnerisch ermittelte Summe aus dem Antriebs- und dem Steuerstrom eingeprägt wird. Es ist aber natürlich auch möglich, den erfindungsgemässen elektromagnetischen Drehantrieb 1 so auszugestalten, dass im Stator 2 zwei getrennten Wicklungssysteme, nämlich eines mit separaten Antriebsspulen und eines mit separaten Steuerspulen, vorgesehen sind. Dann sind beispielsweise auf jedem Längsschenkel 26 jeweils zwei konzentrierte Wicklungen 61a, 61b (Fig. 14, Fig. 15) vorgesehen, von denen eine als Antriebsspule dient und eine als Steuerspule, wie dies im Rahmen des zweiten Ausführungsbeispiels erläutert wird.

Der magnetisch wirksame Kern 31 des Rotors 3 ist beispielsweise so ausgestaltet, wie das insbesondere in Fig. 3 zu erkennen ist, dass er eine Rotorhöhe HR aufweist, welche durch die maximale Erstreckung des magnetisch wirksamen Kerns 31 in axialer Richtung A gegeben. Der magnetisch wirksame Kern 31 ist hier gesamthaft mit einer Höhe in axialer Richtung A ausgestaltet, welche gleich der Rotorhöhe HR ist. Bei dieser Ausgestaltung ist der magnetisch wirksame Kern als Kreisscheibe ausgestaltet, bzw. als Scheibe eines Kreiszylinders, wobei diese Scheibe eine Höhe in axialer Richtung A hat, welche die Rotorhöhe HR ist. Der magnetisch wirksame Kern 31 hat ferner einen Rotordurchmesser RZ, welcher über die gesamte axiale Erstreckung des magnetisch wirksamen Kerns 31 konstant ist. In einem Axialschnitt wie er in Fig. 3 dargestellt ist, hat der magnetisch wirksame Kern 31 also ein rechteckiges Profil, wobei die beiden Seitenlängen des Rechtecks die Rotorhöhe HR und den Rotordurchmesse RZ sind. Der magnetisch wirksame Kern 31 kann natürlich auch als eine Ringscheibe ausgestaltet sein.

Die Querschenkel 27 der Spulenkerne 25 haben eine Statorpolhöhe HS, welche durch die maximale Erstreckung der Stirnflächen 211 der Querschenkel 27 in der axialen Richtung A definiert ist. Vorzugsweise haben alle Stirnflächen 211 in der axialen Richtung A die gleiche Erstreckung, sodass jede Stirnfläche 211 in der axialen Richtung A die gleiche maximale Erstreckung aufweist, nämlich die Statorpolhöhe HS. Ferner ist es bevorzugt, dass jede Stirnfläche 211 so ausgestaltet ist, dass ihre axiale Höhe in Umfangsrichtung gesehen konstant ist. Dann ist die axiale Höhe jeder Stirnfläche 211 gleich der Statorpolhöhe HS.

Bei der in Fig. 3 dargestellten Ausführungsform ist die Statorpolhöhe HS gleich gross wie die Rotorhöhe HR.

Erfindungsgemäss weist jeder Längsschenkel 26 der Spulenkerne 25 einen ersten Abschnitt 261 (siehe insbesondere Fig. 5) und einen zweiten Abschnitt 262 auf, wobei der erste Abschnitt 261 und der zweite Abschnitt 262 bezüglich der axialen Richtung A benachbart zueinander angeordnet sind. Der Querschenkel 27 ist an dem zweiten Abschnitt 262 angeordnet. Für jeden Spulenkern 25 weist die Stirnfläche 211 des Querschenkels 27 von dem ersten Abschnitt 261 des zugehörigen Längsschenkels 26 einen ersten Abstand A1 in radialer Richtung auf, und von dem zweiten Abschnitt 262 einen zweiten Abstand A2 in radialer Richtung, wobei der zweite Abstand A2 grösser ist als der erste Abstand A1. Dies bedeutet, dass jeder Längsschenkel 26 derart ausgestaltet ist, dass der zweite Abschnitt 262 bezüglich des ersten Abschnitts 261 in radialer Richtung nach aussen verschoben ist, sodass sich der für den Rotor 3 zur Verfügung stehende Platz zwischen den Stirnflächen 211 vergrössert, ohne dass dabei die Gefahr eines direkten Übertritts des magnetischen Flusses zwischen dem Längsschenkel 26 und dem magnetisch wirksamen Kern 31 des Rotors 3 besteht. Dadurch, dass die zweiten Abschnitte 262 bezüglich der radialen Richtung und relativ zu den ersten Abschnitten 261 radial nach aussen versetzt sind, vergrössert sich der Abstand, nämlich der zweite Abstand A2, zwischen den Längsschenkeln 26 und den Stirnflächen 211 im Bereich der zweiten Abschnitte 262. Dadurch vergrössert sich auch der Abstand zwischen dem magnetisch wirksamen Kern 31 des Rotors und den Längsschenkeln 26 insbesondere im Bereich der zweiten Abschnitte 262.

Durch die Erweiterung des von den Spulenkernen 25 umschlossenen Raums im Bereich der zweiten Abschnitte 262 wird es somit möglich, einen magnetisch wirksamen Kern 31 mit grösserem Rotordurchmesser RZ zu verwenden, ohne dass dafür der diametrale Abstand zwischen zwei Spulenkernen 25 im Bereich der ersten Enden, also im Bereich des Rückschlusses 22 vergrössert werden muss. Da das in den Rotor 3 einprägbare Drehmoment quadratisch mit dem Rotordurchmesser RZ zunimmt, stellt die erfindungsgemässe Ausgestaltung eine ganz erhebliche Verbesserung dar.

Gemäss einer bevorzugten Ausgestaltung, die insbesondere in den Fig. 3, 5 und 6 zu erkennen ist, sind für jeden Längsschenkel 26 der erste Abschnitt 261 und der zweite Abschnitt 262 parallel zueinander angeordnet, und durch einen Übergangsbereich 263 miteinander verbunden, in welchem sich der radiale Abstand von der zugehörigen Stirnfläche 211 von dem ersten Abstand A1 zu dem zweiten Abstand A2 ändert.. Dieser Übergangsbereich 263 ist vorzugsweise wie der erste Abschnitt 261 und der zweite Abschnitt 262 mit einem rechteckigen Profil ausgestaltet, wobei seine Längsachse mit der axialen Richtung A einen von Null verschiedenen Winkel einschliesst.

Vorzugsweise ist der zweite Abstand A2 (Fig. 5) grösser als die halbe Statorpolhöhe HS, also A2 > HS. Besonders bevorzugt ist es, wenn der zweite Abstand A2 grösser ist als das 1.1-fache des ersten Abstands A1, also A2 > 1.1 A2. Dabei ist es bevorzugt, dass der zweite Abstand A2 grösser ist als das 0.7-fache der Statorpolhöhe HS, also A2 > 0.7 HS.

Ganz besonders bevorzugt ist es, wenn der zweite Abstand A2 grösser ist als das 1.2-fache des ersten Abstands A1, also A2 > 1.2•A2. Dabei ist es bevorzugt, dass der zweite Abstand A2 grösser ist als das 0.8-fache der Statorpolhöhe HS, also A2 > 0.8•HS.

Ganz speziell bevorzugt ist es, wenn der zweite Abstand A2 grösser ist als das 1.25-fache des ersten Abstands A1, also A2 > 1.25•A2. Dabei ist es bevorzugt, dass der zweite Abstand A2 grösser ist als die Statorpolhöhe HS, also A2 > HS.

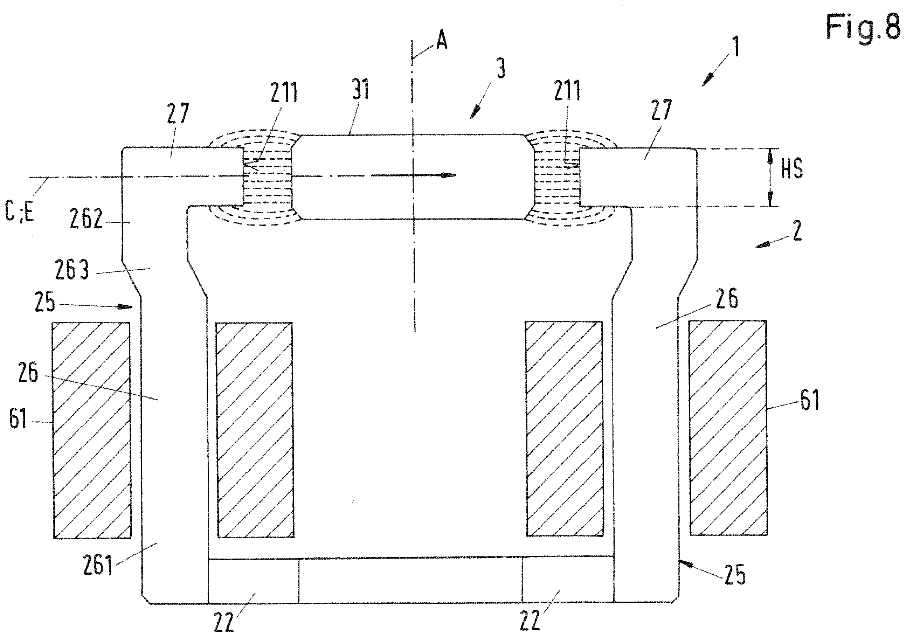

Im Folgenden werden anhand der Fig. 8 bis Fig. 13 verschiedene Varianten für die Ausgestaltung des Rotors 3 bzw. des magnetisch wirksamen Kerns 31 des Rotors 3 erläutert. Dabei wird nur auf die Unterschiede bezüglich der bisherigen Erläuterungen eingegangen. Ansonsten gelten die bisherigen Erläuterungen in gleicher oder in sinngemäss gleicher Weise auch für diese Varianten. Ferner ist es möglich, die anhand der Varianten beschriebenen Massnahmen auch miteinander zu kombinieren.

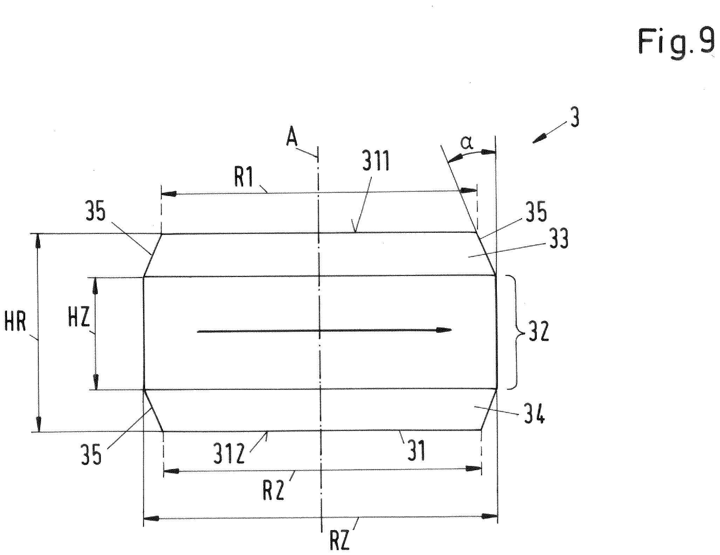

Fig. 8 zeigt in einer der Fig. 3 entsprechenden Darstellung das erste Ausführungsbeispiel des erfindungsgemässen Drehantriebs 1 mit einer ersten Variante für den Rotor 3 bzw. den magnetisch wirksamen Kern 31 des Rotors 3. Zum besseren Verständnis zeigt. Fig. 9 noch diese erste Variant des magnetisch wirksamen Kerns 31.

Um insbesondere die passive magnetische Stabilisierung des Rotors 3 noch zu verbessern, ist bei der ersten Variante der magnetisch wirksame Kern 31 des Rotors 3 so ausgestaltet, dass er eine Rotorhöhe HR aufweist, die grösser ist als die Statorpolhöhe HS.

Der magnetisch wirksame Kern 31 ist bezüglich der axialen Richtung A durch eine erste axiale Begrenzungsfläche 311 und durch eine zweite axiale Begrenzungsfläche 312 begrenzt, die beide senkrecht auf der axialen Richtung A stehen und somit parallel zueinander sind. Der senkrechte Abstand zwischen der ersten axialen Begrenzungsfläche 311 und der zweiten axialen Begrenzungsfläche 312 ist bei dieser Ausgestaltung die Rotorhöhe HR.

Durch die Ausgestaltung des magnetisch wirksamen Kerns 31 des Rotors 3 mit einer Rotorhöhe HR, die grösser ist als die Statorpolhöhe HS, lässt sich die axiale Steifigkeit der magnetischen Lagerung bzw. der magnetischen Stabilisierung des Rotors 3 deutlich verbessern, denn die höhere Ausgestaltung des magnetisch wirksamen Kerns 31 bezüglich der axialen Richtung A führt zu einer Konzentration der magnetischen Flussdichte im Luftspalt zwischen den Stirnflächen 211 der Querschenkel 27 und dem magnetisch wirksamen Kern 31 des Rotors 3. Durch diese Konzentration der Flussdichte im Luftspalt resultiert auch ein deutlich stärkerer Gradient der magnetischen Flussdichte beim Übergang von dem (zumindest näherungsweise) homogenen Feld zwischen den Stirnflächen 211 und dem magnetisch wirksamen Kern 31 in den Bereich des Streufelds, dass bezüglich der axialen Richtung A oberhalb bzw. unterhalb der Querschenkel 27 herrscht. In Fig. 8 sind die Feldlinien des magnetischen Flusses jeweils durch die strichlierten Linien zwischen den Querschenkeln 27 und dem magnetisch wirksamen Kern 31 dargestellt, wobei gerade, parallele Feldlinien den Bereich des homogenen Feldes anzeigen und gekrümmte Feldlinien den Bereich der Streufelder.

Da die axiale Steifigkeit der magnetischen Lagerung quadratisch mit der magnetischen Flussdichte und damit überproportional mit der Rotorhöhe HR ansteigt, lässt sich mit der höheren Ausgestaltung des magnetisch wirksamen Kerns 31 eine deutlich Verbesserung der axialen Steifigkeit der magnetischen Lagerung erzielen. Ferner lässt sich mit dieser Ausgestaltung durch die Konzentration des magnetischen Flusses im Luftspalt auch das Drehmoment vergrössern, welches die Rotation des Rotors 3 antreibt.

Bei der hier beschriebenen Ausgestaltung (siehe Fig. 9) hat der magnetisch wirksamen Kern 31 einen zentralen Bereich 32, welcher bezüglich der axialen Richtung A zwischen einem ersten Randbereich 33 und einem zweiten Randbereich 34 angeordnet ist, wobei beide Randbereiche 33, 34 unmittelbar an den zentralen Bereich 32 angrenzen. Der zentrale Bereich 32 weist einen Durchmesser auf, welches der Rotordurchmesser RZ ist. In axialer Richtung A weist der zentrale Bereich 32 eine zentrale Höhe HZ auf, welche kleiner ist als die Rotorhöhe HR.

Der erste Randbereich 33 bildet die erste axiale Begrenzungsfläche 311 des magnetisch wirksamen Kerns 31, wobei die erste axiale Begrenzungsfläche 311 einen ersten Randdurchmesser R1 aufweist, welches ihr Aussendurchmesser ist. Der zweite Randbereich 34 bildet die zweite axiale Begrenzungsfläche 312 des magnetisch wirksamen Kerns 31, wobei die zweite axiale Begrenzungsfläche 312 einen zweiten Randdurchmesser R2 aufweist, welches ihr Aussendurchmesser ist. Jeder der Randdurchmesser R1 und R2 ist kleiner als der Rotordurchmesser RZ. Dabei können der erste Randdurchmesser R1 und der zweite Randdurchmesser R2 gleich gross sein, so wie dies in Fig. 9 dargestellt ist. In anderen Ausgestaltungen können der erste Randdurchmesser und der zweite Randdurchmesser unterschiedlich gross sein.

Bei der in Fig. 9 dargestellten Ausgestaltung des magnetisch wirksamen Kerns 31 hat der zentrale Bereich 32 in einem Axialschnitt ein rechteckiges Profil, welches die zentrale Höhe HZ als Höhe aufweist und den Rotordurchmesser RZ als Breite.

Besonders bevorzugt ist der zentrale Bereich 32 des magnetisch wirksamen Kerns 31 so ausgestaltet, dass die zentrale Höhe HZ gleich gross ist wie die Statorpolhöhe HS.

Der erste Randbereich 33 und der zweite Randbereich 34 sind jeweils in Form eines Kegelstumpfs ausgestaltet, wobei der Kegelstumpf an seiner Basis jeweils einen Durchmesser aufweist, welcher dem Rotordurchmesser RZ entspricht und an seiner der Basis abgewandten axialen Begrenzungsfläche 311, 312 einen kleineren Durchmesser, welcher dem ersten Randdurchmesser R1 bzw. dem zweiten Randdurchmesser R2 entspricht.

Vorzugsweise - aber nicht notwendigerweise - sind der erste Randbereich 311 und der zweite Randbereich 312 gleichartig ausgestaltet.

Es sind auch solche Ausgestaltungen möglich, bei welchen der erste Randbereich 33 und/oder der zweite Randbereich 34 kreisscheibenförmig mit einem Durchmesser ausgestaltet sind, welcher dem ersten Randdurchmesser R1 bzw. dem zweiten Randdurchmesser R2 entsprechen, sodass dann der erste Randbereich 33 und/oder der zweite Randbereich 34 in einem Axialschnitt ein rechteckiges Profil aufweisen.

Bevorzugt sind jedoch solche Ausgestaltungen des magnetisch wirksamen Kerns 31, bei denen der magnetisch wirksame Kern 31 eine Aussenfläche aufweist, die weder zwischen dem zentralen Bereich 32 und der ersten axialen Begrenzungsfläche 311 noch zwischen dem zentralen Bereich 32 und der zweiten axialen Begrenzungsfläche 312 parallel zur axialen Richtung A verläuft. Es sind also solche Ausgestaltungen bevorzugt, bei welchen sowohl die erste axiale Begrenzungsfläche 311 als auch die zweite axiale Begrenzungsfläche durch Übergänge 35 mit dem zentralen Bereich 32 verbunden sind, die schräg zur axialen Richtung A oder gekrümmt verlaufen.

Durch diese Massnahme wird die Kippsteifigkeit des Rotors 3, das heisst sein Widerstand gegen Verkippungen, bzw. seine Fähigkeit aus einer verkippten Position in die Soll-Position zurückzukehren, deutlich verbessert.

Wie bereits erwähnt, sind bei der in Fig. 9 dargestellten Ausgestaltung der erste Randbereich 33 und der zweite Randbereich 34 jeweils in Form eines Kegelstumpfs ausgestaltet. Die Neigung des Kegelstumpfs wird dabei durch einen Kegelstumpfwinkel α beschrieben, welcher durch den spitzen Winkel zwischen dem Übergang 35 und der axialen Richtung A gegeben ist.

In der Praxis haben sich bestimmte Kombinationen der geometrischen Abmessungen als besonders vorteilhaft erwiesen.

Für das Höhenverhältnis aus der Rotorhöhe HR und der zentralen Höhe HZ ist der Bereich von 1.2 bis 1.6 bevorzugt, also 1.2 ≤ HR/HZ ≤ 1.6, wobei der Kegelstumpfwinkel α zwischen 15 Grad und 60 Grad ist, also 15° ≤ α ≤ 60°. Dabei hat es sich als vorteilhaft erwiesen, wenn der Kegelstumpfwinkel α umso grösser ist, je grösser das Höhenverhältnis HR/HZ ist.

Ferner hat es sich als vorteilhaft erwiesen, wenn das Verhältnis aus dem Rotordurchmesser RZ und der Rotorhöhe HR zwischen zwei und drei liegt, also 2 ≤ RZ/HR ≤ 3, wobei dieses Verhältnis vorzugsweise umso kleiner gewählt werden kann, desto grösser der Kegelstumpfwinkel α ist.

Besonders bevorzugt ist das Höhenverhältnis aus der Rotorhöhe HR und der zentralen Höhe HZ im Bereich von 1.3 bis 1.5, also 1.3 ≤ HR/HZ ≤ 1.5, wobei der Kegelstumpfwinkel α zwischen 20 Grad und 30 Grad ist, also 20° ≤ α ≤ 30°. Für das Verhältnis aus dem Rotordurchmesser RZ und der Rotorhöhe HR ist der Bereich von 2.3 bis 2.7 besonders bevorzugt, also 2.3 ≤ RZ/HR ≤ 2.7.

Speziell bevorzugt ist das Höhenverhältnis aus der Rotorhöhe HR und der zentralen Höhe HZ etwa 1.46, also HR/HZ = 1.46, wobei der Kegelstumpfwinkel α etwa 22.5 Grad ist, also α = 22.5°.

Falls der magnetisch wirksamen Kern 31 des Rotors 3 in eine verkippte Stellung gerät, bei welcher die magnetische Mittelebene C mit der axialen Richtung A einen von Null verschiedenen Winkel einschliesst, wirkt beispielsweise in dem Bereich des magnetisch wirksamen Kerns 31, welcher der darstellungsgemäss linken Stirnfläche 211 (Fig. 8) gegenüberliegt, in axialer Richtung A eine Kraftkomponente, die entgegengesetzt gerichtet ist zu einer Kraftkomponente in axialer Richtung A, die in dem Bereich des magnetisch wirksamen Kerns 31 wirkt, welcher der darstellungsgemäss rechten Stirnfläche 211 gegenüberliegt. Diese beiden Kraftkomponenten in axialer Richtung A üben somit ein Drehmoment auf den magnetisch wirksamen Kern 31 aus, welches diesen zurück in seine Soll-Position, also in eine unverkippte Position bringt, in welcher die magnetische Mittelebene C senkrecht auf der axialen Richtung A steht.

Falls der magnetisch wirksamen Kern 31 des Rotors 3 in eine bezüglich der axialen Richtung A verschobene bzw. ausgelenkte Position gerät, steht die magnetische Mittelebene C zwar noch senkrecht auf der axialen Richtung A, ist aber bezüglich der radialen Ebene E parallel nach unten (darstellungsgemäss) oder nach oben verschoben. In dieser Position wirkt in dem Bereich des magnetisch wirksamen Kerns 31, welcher der darstellungsgemäss linken Stirnfläche 211 gegenüberliegt, in axialer Richtung A eine Kraftkomponente, die gleich gerichtet ist wie eine Kraftkomponente in axialer Richtung A, die in dem Bereich des magnetisch wirksamen Kerns 31 wirkt, welcher der darstellungsgemäss rechten Stirnfläche 211 gegenüberliegt. Diese beiden Kraftkomponenten üben somit ein Kraft auf den magnetisch wirksamen Kern 31 aus, welche diesen zurück in seine Soll-Position bringt, in welcher die magnetische Mittelebene C in der radialen Ebene E liegt.

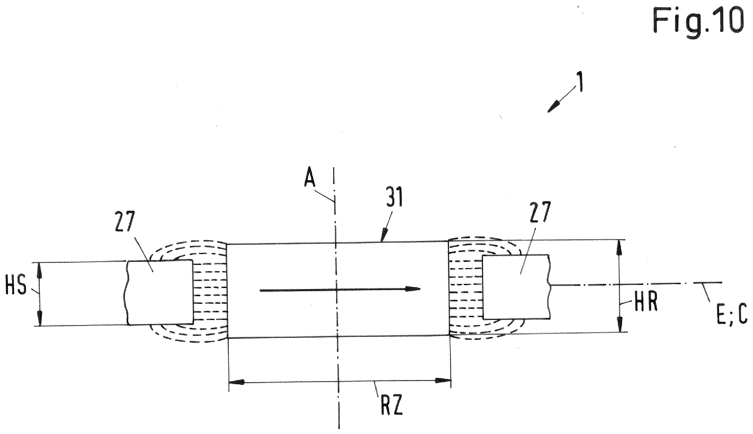

Fig. 10 veranschaulicht eine zweite Variante für den magnetisch wirksamen Kern 31 des Rotors 3. Bei dieser ersten Variante ist der magnetisch wirksame Kern 31 gesamthaft mit einer Höhe in axialer Richtung A ausgestaltet, welche gleich der Rotorhöhe HR ist. Wiederum ist dabei die Rotorhöhe HR grösser als die Statorpolhöhe HS. Im Unterschied zu der ersten Variante hat bei dieser Ausgestaltung gemäss Fig. 10 der magnetisch wirksame Kern 31 keine ausgeprägten Randbereiche, die von einem zentralen Bereich unterscheidbar wären. Der magnetisch wirksame Kern 31 ist als Kreisscheibe ausgestaltet, bzw. als Scheibe eines Kreiszylinders, wobei diese Scheibe den Durchmesser hat, welcher der Rotordurchmesser RZ ist, und eine Höhe in axialer Richtung, welches die Rotorhöhe HR ist. Der magnetisch wirksame Kern 31 kann natürlich auch als eine Ringscheibe ausgestaltet sein.

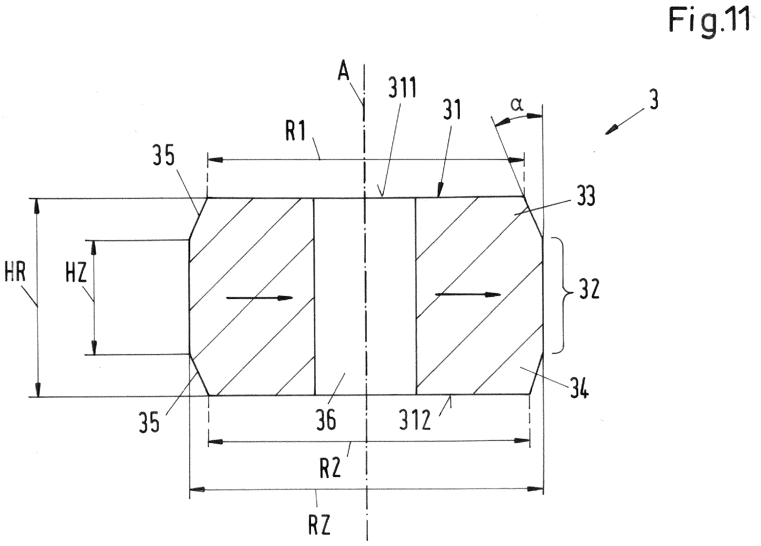

Fig. 11 zeigt eine dritte Variante für den magnetisch wirksamen Kern 31 in einem Schnitt in axialer Richtung. Die dritte Variante entspricht weitgehend der in Fig. 9 dargestellten ersten Variante, weist jedoch eine zentrale Bohrung 36 auf, die sich in axialer Richtung A durch den gesamten magnetisch wirksamen Kern 31 von der ersten axialen Begrenzungsfläche 311 bis zu der zweiten axialen Begrenzungsfläche 312 erstreckt.

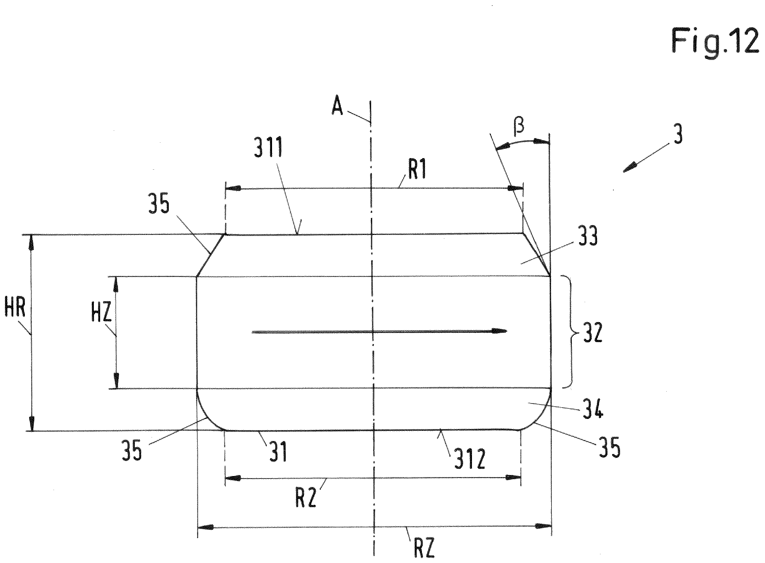

Fig. 12 zeigt eine vierte Variante für den magnetisch wirksamen Kern 31. Die vierte Variante entspricht weitgehend der in Fig. 9 dargestellten ersten Variante, unterscheidet sich jedoch durch die Ausgestaltung des ersten Randbereichs 33 und des zweiten Randbereichs 34. Bei der dritten Variante sind beide Randbereiche 33, 34 jeweils in Form einer Kugelscheibe oder in Form einer Paraboloidscheibe ausgestaltet, das heisst, die Übergänge 35 sind hierbei jeweils gekrümmt ausgestaltet, beispielsweise als Teil einer Kugelschale oder als Teil eines Paraboloids. Die Neigung der Übergänge 35 kann beispielsweise durch einen Neigungswinkel β beschrieben werden, der in sinngemäss gleicher Weise wie der Kegelstumpfwinkel α festgelegt werden kann. So ist der Neigungswinkel β beispielsweise der spitze Winkel, welchen eine Tangente an den Übergang 35 mit der axialen Richtung A einschlicht. Für den Neigungswinkel β gelten sinngemäss die gleichen Erläuterungen wie für den Kegelstumpfwinkel α.

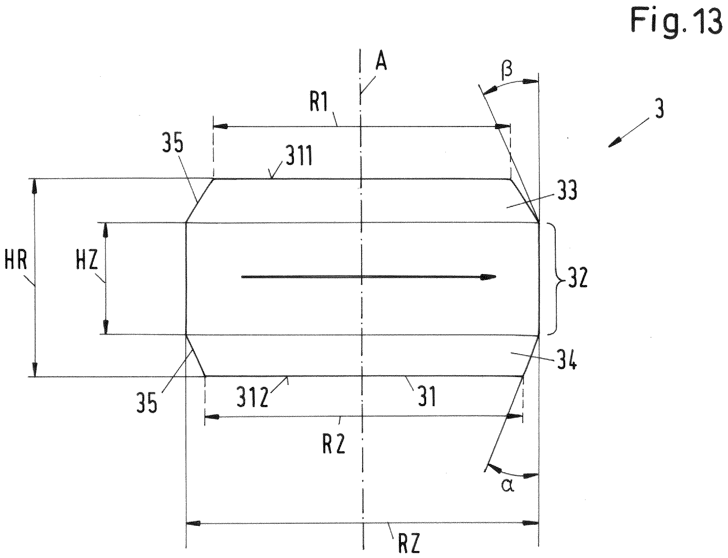

Fig. 13 zeigt eine fünfte Variante für den magnetisch wirksamen Kern 31. Die fünfte Variante entspricht weitgehend der in Fig. 9 dargestellten ersten Variante, unterscheidet sich jedoch durch die Ausgestaltung des ersten Randbereichs 33. Bei der vierten Variante ist nur der zweite Randbereich 34 in Form eines Kegelstumpfs ausgestaltet, während der erste Randbereich in Form einer Kugelscheibe oder in Form einer Paraboloidscheibe ausgestaltet ist.

Fig. 14 zeigt in einer perspektivischen Darstellung ein zweites Ausführungsbeispiel eines erfindungsgemässen elektromagnetischen Drehantriebs 1. Zum besseren Verständnis zeigt Fig. 15 das zweite Ausführungsbeispiel noch in einem Schnitt in axialer Richtung A.

Im Folgenden wird nur auf die Unterschiede zu dem ersten Ausführungsbeispiel eingegangen. Gleiche Teile oder von der Funktion her gleichwertige Teile des zweiten Ausführungsbeispiels sind mit den gleichen Bezugszeichen bezeichnet wie bei dem ersten Ausführungsbeispiel bzw. seinen Varianten. Insbesondere haben die Bezugszeichen die gleiche Bedeutung wie sie bereits im Zusammenhang mit dem ersten Ausführungsbeispielen erläutert sind. Es versteht sich, dass alle vorangehenden Erläuterungen des ersten Ausführungsbeispiels und seiner Varianten in gleicher Weise oder in sinngemäss gleicher Weise auch für das zweite Ausführungsbeispiel gelten.

Bei dem zweiten Ausführungsbeispiel ist der Stator 2 des elektromagnetischen Drehantriebs 1 derart ausgestaltet, dass an jedem Längsschenkel 26 zwei konzentrierte Wicklungen 61a, 61b vorgesehen sind, von denen jede den jeweiligen Längsschenkel 26 umgibt, und die bezüglich der axialen Richtung A benachbart zueinander angeordnet sind.

Diese Ausführungsform eignet sich insbesondere für Ausgestaltungen nach dem Prinzip des lagerlosen Motors, bei welchen zur Erzeugung des Antriebsfelds und des Steuerfelds separate Wicklungssysteme vorgesehen sind. So kann beispielsweise eine konzentrierte Wicklung 61a als Antriebsspule 61a eingesetzt werden, während die andere konzentrierte Wicklung 61b als Steuerspule 61b eingesetzt werden. Mit der Gesamtheit aller Antriebsspulen 61a wird dann das Antriebsfeld generiert, welches ein elektromagnetisches Drehfeld ist, während mit der Gesamtheit der Steuerspulen 61b das Steuerfeld generiert wird, welches ebenfalls ein elektromagnetisches Drehfeld ist, und welches dem Antriebsfeld überlagert wird. Durch die Überlagerung des Antriebsfelds und des Steuerfelds lassen sich dann die Tangentialkräfte auf den Rotor 3 generieren, welche das Drehmoment zum Antreiben der Rotation erzeugen, sowie eine beliebig einstellbare Querkraft in der radialen Ebene E, mit welcher die Position des magnetisch wirksamen Kerns 31 in der radialen Ebene aktiv regelbar ist.

Je nach Anwendungsfall kann die Ausgestaltung mit separaten Antriebsspulen 61a und separaten Steuerspulen 61b die Ansteuerung bzw. die Regelung des elektromagnetischen Drehantriebs vereinfachen.

Die auf dem gleichen Längsschenkel 26 axial zueinander benachbart angeordneten beiden konzentrierten Wicklungen, nämlich die Antriebsspule 61a und die Steuerspule 61b sollen sich nicht berühren. Dies kann in vorteilhafter Weise so realisiert werden, dass bezüglich der axialen Richtung A zwischen der Antriebsspule 61a und der Steuerspule 61b eine Kontrolleinrichtung 62 oder ein Teil einer Kontrolleinrichtung 62 zur Ansteuerung und zur Regelung des elektromagnetischen Drehantriebs 1 angeordnet ist. Diese Kontrolleinrichtung 62 kann beispielsweise auch Leistungselektronik für die konzentrierten Wicklungen 61a, 61b umfassen. Insbesondere kann diese Kontrolleinrichtung 62 zur Ansteuerung und zur Regelung der Steuerspulen 61b dienen. Vorzugsweise ist die zwischen den beiden konzentrierten Wicklungen angeordnete Kontrolleinrichtung als Platine bzw. PCB (printed circuit board) ausgestaltet.

Durch die Erfindung wird ferner eine Zentrifugalpumpe 100 zum Fördern eines Fluids vorgeschlagen, die dadurch gekennzeichnet ist, dass die Zentrifugalpumpe 100 einen elektromagnetischen Drehantrieb 1 umfasst, der erfindungsgemäss ausgestaltet ist, wobei der Rotor 3 des elektromagnetischen Drehantriebs 1 als Rotor 3 der Zentrifugalpumpe 100 ausgestaltet ist.

Fig. 16 zeigt ein Ausführungsbeispiel einer erfindungsgemässen Zentrifugalpumpe, die gesamthaft mit dem Bezugszeichen 100 bezeichnet ist, in einem Schnitt in axialer Richtung A.

Die Zentrifugalpumpe 100 umfasst eine Pumpeneinheit 50 mit einem Pumpengehäuse 51, das einen Einlass 52 und einen Auslass 53 für das zu fördernde Fluid umfasst, wobei der Rotor 3 im Pumpengehäuse 51 angeordnet ist, und eine Mehrzahl von Flügeln 54 zum Fördern des Fluids umfasst. Die Pumpeneinheit 50 ist derart ausgestaltet, dass die Pumpeneinheit 50 so in den Stator 2 einsetzbar ist, dass der magnetisch wirksame Kern des Rotors 31 von den Stirnflächen 211 der Querschenkel 27 umgeben wird.

Ein vorteilhafter Aspekt ist es, dass der Rotor 3 als Integralrotor ausgestaltet ist, weil er sowohl der Rotor 3 des elektromagnetischen Drehantriebs 1 ist, als auch der Rotor 3 der Zentrifugalpumpe 100, mit welchem das Fluid gefördert wird. Insgesamt erfüllt der Rotor 3 somit drei Funktionen in einem: Er ist der Rotor 3 des elektromagnetischen Antriebs 1, er ist der Rotor 3 der magnetischen Lagerung, und er ist das Laufrad, mit welchem auf das Fluid bzw. die Fluide eingewirkt wird. Diese Ausgestaltung als Integralrotor bietet den Vorteil einer sehr kompakten und platzsparenden Ausgestaltung.

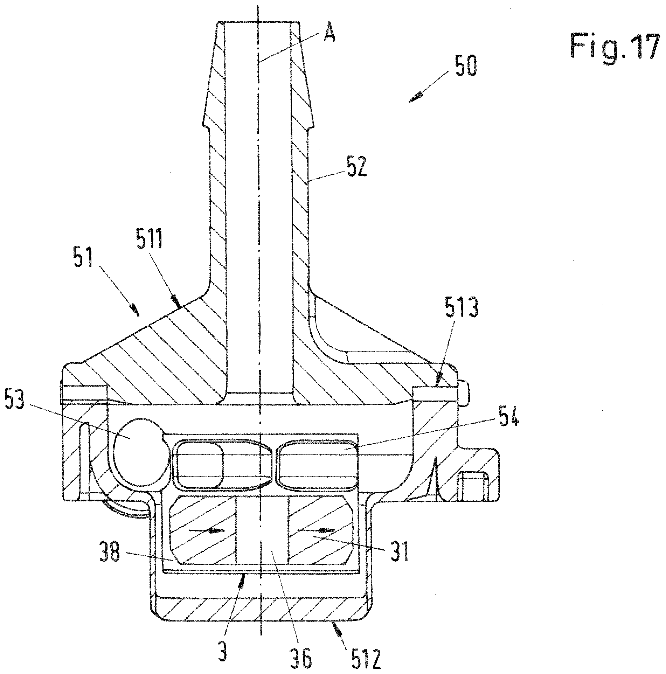

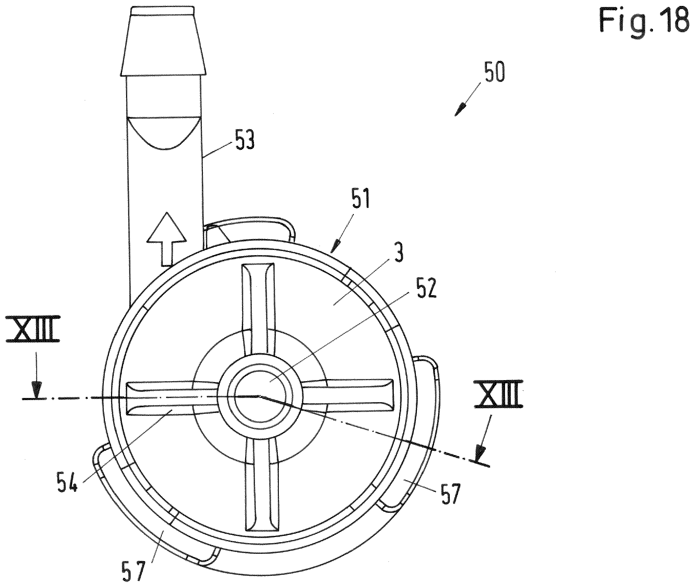

Zum besseren Verständnis zeigt Fig. 17 noch eine etwas detailliertere Darstellung der Pumpeneinheit 50 der Zentrifugalpumpe 100 in einem Schnitt entlang der Schnittlinie XVII-XVII in Fig. 18. Fig. 18 zeigt eine Aufsicht auf die Pumpeneinheit 50 aus der axialen Richtung A.

Das Pumpengehäuse 51 der Pumpeneinheit umfasst ein Basisteil 512 und einen Deckel 511, welche dichtend miteinander verbunden sind, wobei der Auslass 53 des Pumpengehäuses 51 vollständig im Basisteil 512 angeordnet ist. Der Deckel 511 umfasst den Einlass 52, welcher sich in axialer Richtung A erstreckt, sodass das Fluid den Rotor 3 aus der axialen Richtung A anströmt.

Ein wesentlicher Aspekt ist hierbei auch, dass der Auslass 53 vollständig im Basisteil 512 angeordnet ist, sodass der Auslass 53 keine Trennfugen, Schweissnähte oder ähnliche Verbindungsstellen aufweist.

Zum dichtenden Verbinden des Deckels 511 und des Basisteils 512 sind alle an sich bekannten Methoden geeignet. So können das Basisteil 512 und der Deckel 511 beispielsweise durch eine Verschraubung oder durch eine Klickverbindung oder durch eine Einrastverbindung, durch Verkleben oder durch verschiedene Arten von Verschweissungen, beispielsweise durch Infrarotschweissen, miteinander verbunden werden. Je nach Art der Verbindung kann es vorteilhaft sein, zwischen dem Basisteil 512 und dem Deckel 511 ein Dichtungselement 513, beispielsweise einen O-Ring vorzusehen.

Der Rotor 3 umfasst die Mehrzahl von Flügeln 54 zum Fördern des Fluids. Bei dem hier beschriebenen Ausführungsbeispiel sind insgesamt vier Flügel 54 vorgesehen, wobei diese Anzahl beispielhaften Charakter hat. Der Rotor 3 umfasst ferner eine Ummantelung 38, mit welcher der magnetisch wirksame Kern 31 des Rotors 3 umschlossen und vorzugsweise hermetisch eingekapselt ist, sodass der magnetisch wirksame Kern 31 des Rotors 3 nicht in Kontakt mit dem zu fördernden Fluid kommt. Alle Flügel 54 sind auf der Ummantelung 38 angeordnet und bezüglich der Umfangsrichtung des Rotors 3 äquidistant angeordnet. Jeder Flügel 54 erstreckt sich in radialer Richtung nach aussen und ist drehfest mit dem Ummantelung 38 verbunden. Die Flügel 54 können separate Komponenten sein, die dann auf der Ummantelung 38 fixiert werden. Es ist natürlich auch möglich, dass alle Flügel 54 integraler Bestandteil der Ummantelung 38 sind, dass also die Ummantelung 38 mit allen Flügeln 54 einstückig ausgestaltet ist. Der Rotor 3 mit den Flügeln 54 bildet das Flügelrad bzw. das Laufrad der Zentrifugalpumpe 100, mit welchem auf das Fluid oder die Fluide eingewirkt wird.

Vorzugsweise umfasst der Rotor 3 die zentrale Bohrung 36, welche sich in axialer Richtung A vollständig durch den Rotor 3 hindurch erstreckt. Durch diese zentrale Bohrung 36 lässt sich ein zumindest teilweiser Axialschubausgleich gewährleisten, sodass die passive magnetische axiale Lagerung des Rotors 3 entlastet wird.

Je nach Anwendungsfall, beispielsweise, wenn die Zentrifugalpumpe als Blutpumpe eingesetzt wird, ist es bevorzugt, wenn das Pumpengehäuse 51 der Pumpeneinheit 50 sowie die Ummantelung 38 und die Flügel 54 aus einem oder mehreren Kunststoffen hergestellt wird. Geeignete Kunststoffe sind: Polyethylene (PE), Low Density Polyethylene (LDPE), Ultra Low Density Polyethylene (ULDPE), Ethylene Vinyl Acetate (EVA), Polyethylene Terephthalate (PET), Polyvinylchlorid (PVC), Polypropylene (PP), Polyurethan (PU), Polyvinylidene Fluoride (PVDF), Acrylonitrile Butadiene Styrene (ABS), Polyacryl, Polycarbonate (PC), Polyetheretherketon (PEEK) oder Silicone. Für viele Anwendungen sind auch die unter dem Markennamen Teflon bekannten Materialien Polytetrafluoroethylene (PTFE) und und Perfluoralkoxy-Polymere (PFA) als Kunststoff geeignet.

Vorzugsweise ist die Pumpeneinheit 50 zum lösbaren Verbinden mit dem Stator 2 der Zentrifugalpumpe 100 ausgestaltet ist. Hierzu können an dem Pumpengehäuse 51 beispielsweise mehrere Laschen 57 vorgesehen sein, die mit dem Stator 2 in Form einer Bajonettverbindung zusammenwirken können.

In einer besonders bevorzugten Ausführungsform ist die Pumpeneinheit 50 als Einmalvorrichtung für den Einmalgebrauch ausgestaltet, die in den als wiederverwendbare Vorrichtung ausgestalteten Stator 2 einsetzbar ist. Die Zentrifugalpumpe 100 setzt sich dann aus der Pumpeneinheit 50 zusammen, die als Einmalvorrichtung für den Einmalgebrauch ausgestaltet ist, sowie dem Stator 2, der als wiederverwendbare Vorrichtung ausgestaltet ist, die für den Mehrfachgebrauch ausgebildet ist. Der Stator 2 umfasst dabei typischerweise auch die Kontroll-, Regel- und Versorgungseinheiten des elektromagnetischen Drehantriebs 1.

Mit dem Begriff "Einmalvorrichtung" und anderen Zusammensetzungen mit dem Bestandteil "Einmal", sind dabei solche Komponenten bzw. Teile gemeint, die für den Einmalgebrauch ausgestaltet sind, die also bestimmungsgemäss nur ein einziges Mal benutzt werden können und dann entsorgt werden. Für eine neue Anwendung muss dann ein neues, bisher unbenutztes Einmalteil eingesetzt werden. Bei der Konzipierung bzw. der Ausgestaltung der Einmalvorrichtung ist es daher ein wesentlicher Aspekt, dass die Einmalvorrichtung in möglichst einfacher Weise mit der wiederverwendbaren Vorrichtung zu der Zentrifugalpumpe zusammenfügbar ist. Die Einmalvorrichtung soll also in sehr einfacher Weise ersetzt werden können, ohne dass dafür ein hoher Montageaufwand notwendig ist. Besonders bevorzugt soll die Einmalvorrichtung ohne die Verwendung von Werkzeugen mit der wiederverwendbaren Vorrichtung zusammenfügbar und von dieser trennbar sein. Die Pumpeneinheit 50 kann als eine solche Einmalvorrichtung ausgestaltet werden.

Die Zentrifugalpumpe 100 kann beispielsweise in der Medizinaltechnik als Blutpumpe eingesetzt werden oder in der pharmazeutischen Industrie oder in der biotechnologischen Industrie Verwendung finden. Speziell eignet sich die Zentrifugalpumpe 100 für solche Anwendungen, bei denen ein sehr hohes Mass an Reinheit oder Sterilität derjenigen Komponenten wesentlich ist, die mit den zu mischenden Substanzen in Kontakt kommen.

## Patentansprüche

1. Elektromagnetischer Drehantrieb, der als Tempelmotor ausgestaltet ist, mit einem Rotor (3), der einen ring- oder scheibenförmigen magnetisch wirksamen Kern (31) umfasst, sowie mit einem Stator (2), welcher als Lager- und Antriebsstator ausgestaltet ist, mit welchem der Rotor (3) im Betriebszustand berührungslos magnetisch um eine Solldrehachse antreibbar ist, die eine axiale Richtung (A) definiert, und mit welchem der Rotor (3) berührungslos magnetisch bezüglich des Stators (2) lagerbar ist, wobei der Rotor (3) in einer zur axialen Richtung (A) senkrechten radialen Ebene (E) aktiv magnetisch gelagert ist, und in axialer Richtung (A) sowie gegen Verkippungen passiv magnetisch stabilisiert ist, wobei der Stator (2) eine Mehrzahl von Spulenkernen (25) aufweist, von denen jeder einen Längsschenkel (26) umfasst, welcher sich in axialer Richtung (A) erstreckt, sowie einen in der radialen Ebene (E) angeordneten Querschenkel (27), welcher sich von dem Längsschenkel (26) in einer radialen Richtung erstreckt, und durch eine Stirnfläche (211) begrenzt wird, welche dem magnetisch wirksamen Kern (31) des Rotors (3) zugewandt ist, und wobei an jedem Längsschenkel (26) mindestens eine konzentrierte Wicklung (61) angeordnet ist, welche den jeweiligen Längsschenkel (26) umgibt, wobei jeder Längsschenkel (26) einen ersten Abschnitt (261) und einen zweiten Abschnitt (262) umfasst, welche bezüglich der axialen Richtung (A) benachbart zueinander angeordnet sind, wobei der Querschenkel (27) an dem zweiten Abschnitt (262) angeordnet ist, wobei die Stirnfläche (211) des Querschenkels (27) von dem ersten Abschnitt (261) des zugehörigen Längsschenkels (26) einen ersten Abstand (A) in radialer Richtung aufweist, und von dem zweiten Abschnitt (262) einen zweiten Abstand (A2) in radialer Richtung, **dadurch gekennzeichnet dass** der zweite Abstand (A2) grösser ist als der erste Abstand (A1).

2. Drehantrieb nach Anspruch 1, wobei für jeden Längsschenkel (26) der erste Abschnitt (261) und der zweite Abschnitt (262) parallel zueinander angeordnet sind, und durch einen Übergangsbereich (263) miteinander verbunden sind, in welchem sich der radiale Abstand von der zugehörigen Stirnfläche (211) von dem ersten Abstand (A1) zu dem zweiten Abstand (A2) ändert.

3. Drehantrieb nach einem der vorangehenden Ansprüche, wobei der magnetisch wirksame Kern (31) des Rotors (3) eine Rotorhöhe (HR) aufweist, welche die maximale Erstreckung des magnetisch wirksamen Kerns (31) in axialer Richtung (A) ist, und wobei die Rotorhöhe (HR) grösser ist als eine Statorpolhöhe (HS), welche durch die maximale Erstreckung der Stirnflächen (211) der Querschenkel in axialer Richtung (A) festgelegt ist.

4. Drehantrieb nach einem der vorangehenden Ansprüche, wobei der zweite Abstand (A2) grösser ist als die Hälfte einer Statorpolhöhe (HS), welche durch die maximale Erstreckung der Stirnflächen (211) der Querschenkel (27) in axialer Richtung (A) festgelegt ist.

5. Drehantrieb nach einem der Ansprüche 3-5, wobei der magnetisch wirksame Kern (31) einen zentralen Bereich (32) umfasst, welcher bezüglich der axialen Richtung (A) zwischen einem ersten Randbereich (33) und einem zweiten Randbereich (34) angeordnet ist, und welcher einen Rotordurchmesser (RZ) aufweist, wobei der erste Randbereich (33) eine erste axiale Begrenzungsfläche (311) des magnetisch wirksamen Kerns (31) bildet, die einen ersten Randdurchmesser (R1) aufweist, wobei der zweite Randbereich (34) eine zweite axiale Begrenzungsfläche (312) des magnetisch wirksamen Kerns bildet (31), die einen zweiten Randdurchmesser (R2) aufweist, und wobei jeder Randdurchmesser (R1, R2) kleiner ist als der Rotordurchmesser (RZ).

6. Drehantrieb nach Anspruch 5, wobei der zentrale Bereich (32) eine zentrale Höhe (HZ) aufweist, welches die Erstreckung des zentralen Bereichs (32) in axialer Richtung (A) ist, wobei die zentrale Höhe (HZ) gleich gross ist wie die Statorpolhöhe (HS).

7. Drehantrieb nach einem der Ansprüche 5-6, wobei der magnetisch wirksame Kern (31) eine Aussenfläche aufweist, die weder zwischen dem zentralen Bereich (32) und der ersten axialen Begrenzungsfläche (311) noch zwischen dem zentralen Bereich (32) und der zweiten axialen Begrenzungsfläche (312) parallel zur axialen Richtung (A) verläuft.

8. Drehantrieb nach einem der vorangehenden Ansprüche, wobei an jedem Längsschenkel zwei konzentrierte Wicklungen (61a, 61b) vorgesehen sind, von denen jede den jeweiligen Längsschenkel (26) umgibt, und die bezüglich der axialen Richtung (A) benachbart zueinander angeordnet sind.

9. Zentrifugalpumpe zum Fördern eines Fluids, **dadurch gekennzeichnet, dass** die Zentrifugalpumpe einen elektromagnetischen Drehantrieb (1) umfasst, der gemäss einem der vorangehenden Ansprüche ausgestaltet ist, wobei der Rotor (3) des elektromagnetischen Drehantriebs (1) als Rotor (3) der Zentrifugalpumpe ausgestaltet ist.

10. Zentrifugalpumpe nach Anspruch 9, welche eine Pumpeneinheit (50) mit einem Pumpengehäuse (51) umfasst, das einen Einlass (52) und einen Auslass (53) für das zu fördernde Fluid umfasst, wobei der Rotor (3) im Pumpengehäuse (51) angeordnet ist, und eine Mehrzahl von Flügeln (54) zum Fördern des Fluids umfasst, wobei die Pumpeneinheit (50) derart ausgestaltet ist, dass die Pumpeneinheit (50) so in den Stator (2) einsetzbar ist, dass der magnetisch wirksame Kern (31) des Rotors (3) von den Stirnflächen (211) der Querschenkel (27) umgeben wird.

11. Pumpeneinheit für eine Zentrifugalpumpe, **dadurch gekennzeichnet, dass** die Pumpeneinheit (50) für eine Zentrifugalpumpe (100) ausgestaltet ist, welche nach Anspruch 10 ausgestaltet ist.

12. Pumpeneinheit nach Anspruch 11, wobei das Pumpengehäuse (51) ein Basisteil (512) und einen Deckel (511) umfasst, welche dichtend miteinander verbunden sind, wobei der Auslass (53) des Pumpengehäuses (51) vollständig im Basisteil (512) angeordnet ist.

13. Pumpeneinheit nach einem der Ansprüche 11-12, wobei der Rotor (3) eine zentrale Bohrung (36) aufweist, welche sich in axialer Richtung (A) vollständig durch den Rotor (3) hindurch erstreckt.

14. Pumpeneinheit nach einem der Ansprüche 11-13, welche zum lösbaren Verbinden mit dem Stator (2) der Zentrifugalpumpe (100) nach Anspruch 9 ausgestaltet ist.

15. Pumpeneinheit nach einem der Ansprüche 11-14, wobei die Pumpeneinheit (50) als Einmalvorrichtung für den Einmalgebrauch ausgestaltet ist.

## Claims

1. An electromagnetic rotary drive designed as a temple motor, with a rotor (3) comprising a ring-shaped or disk-shaped magnetically effective core (31), and with a stator (2) designed as a bearing and drive stator, by means of which the rotor (3) can be magnetically driven without contact in the operating state about a desired axis of rotation which defines an axial direction (A), and by means of which the rotor (3) can be magnetically levitated without contact with respect to the stator (2), wherein the rotor (3) is actively magnetically levitated in a radial plane (E) perpendicular to the axial direction (A) and is passively magnetically stabilized in the axial direction (A) and against tilting, wherein the stator (2) has a plurality of coil cores (25), each of which comprises a longitudinal limb (26) extending in the axial direction (A) and a transverse limb (27) arranged in the radial plane (E), which extends from the longitudinal limb (26) in a radial direction and is bounded by an end face (211) facing the magnetically effective core (31) of the rotor (3), and wherein at least one concentrated winding (61) is arranged on each longitudinal limb (26) which surrounds the respective longitudinal limb (26), wherein each longitudinal limb (26) comprises a first portion (261) and a second portion (262) which are arranged adjacent to each other with respect to the axial direction (A), wherein the transverse limb (27) is arranged at the second portion (262), wherein the end face (211) of the transverse limb (27) has a first distance (A) in the radial direction from the first portion (261) of the associated longitudinal limb (26) and a second distance (A2) in the radial direction from the second portion (262), **characterized in that** the second distance (A2) is greater than the first distance (A1).

2. A rotary drive according to claim 1, wherein for each longitudinal limb (26) the first portion (261) and the second portion (262) are arranged parallel to each other and are connected to each other by a transition region (263) in which the radial distance from the associated end face (211) changes from the first distance (A1) to the second distance (A2).

3. A rotary drive according to anyone of the preceding claims, wherein the magnetically effective core (31) of the rotor (3) has a rotor height (HR) which is the maximum extension of the magnetically effective core (31) in the axial direction (A), and wherein the rotor height (HR) is greater than a stator pole height (HS) which is defined by the maximum extension of the end faces (211) of the transverse limbs in the axial direction (A).

4. A rotary drive according to anyone of the preceding claims, wherein the second distance (A2) is greater than half of a stator pole height (HS), which is defined by the maximum extension of the end faces (211) of the transverse limbs (27) in the axial direction (A).

5. A rotary drive according to anyone of the claims 3 to 5, wherein the magnetically effective core (31) comprises a central region (32) which is arranged with respect to the axial direction (A) between a first edge region (33) and a second edge region (34), and which has a rotor diameter (RZ), wherein the first edge region (33) forms a first axial boundary surface (311) of the magnetically effective core (31) which has a first edge diameter (R1), wherein the second edge region (34) forms a second axial boundary surface (312) of the magnetically effective core (31) which has a second edge diameter (R2), and wherein each edge diameter (R1, R2) is smaller than the rotor diameter (RZ).

6. A rotary drive according to claim 5, wherein the central region (32) has a central height (HZ) which is the extension of the central region (32) in the axial direction (A), wherein the central height (HZ) is the same size as the stator pole height (HS).

7. A rotary drive according to anyone of the claims 5 to 6, wherein the magnetically effective core (31) has an outer surface that is not parallel to the axial direction (A) either between the central region (32) and the first axial boundary surface (311) or between the central region (32) and the second axial boundary surface (312).

8. A rotary drive according to anyone of the preceding claims, wherein two concentrated windings (61a, 61b) are provided on each longitudinal limb, each of which surrounds the respective longitudinal limb (26), and which are arranged adjacent to each other with respect to the axial direction (A).

9. A centrifugal pump for conveying a fluid, **characterized in that** the centrifugal pump comprises an electromagnetic rotary drive (1) which is designed according to anyone of the preceding claims, the rotor (3) of the electromagnetic rotary drive (1) being designed as the rotor (3) of the centrifugal pump.

10. A centrifugal pump according to claim 9, which comprises a pump unit (50) with a pump housing (51) comprising an inlet (52) and an outlet (53) for the fluid to be conveyed, wherein the rotor (3) is arranged in the pump housing (51) and comprises a plurality of vanes (54) for conveying the fluid, wherein the pump unit (50) is designed in such a way that the pump unit (50) can be inserted into the stator (2) such that the magnetically effective core (31) of the rotor (3) is surrounded by the end faces (211) of the transverse limbs (27).

11. A pump unit for a centrifugal pump, **characterized in that** the pump unit (50) is designed for a centrifugal pump (100), which is designed according to claim 10.

12. A pump unit according to claim 11, wherein the pump housing (51) comprises a base part (512) and a cover (511) which are connected to each other in a sealing manner, wherein the outlet (53) of the pump housing (51) is completely arranged in the base part (512).

13. A pump unit according to anyone of the claims 11 to 12, wherein the rotor (3) has a central bore (36) extending completely through the rotor (3) in the axial direction (A).

14. A pump unit according to anyone of the claims 11 to 13, which is designed for detachable connection to the stator (2) of the centrifugal pump (100) according to claim 9.

15. A pump unit according to anyone of the claims 11 to 14, wherein the pump unit (50) is designed as a single-use device for single use.

## Revendications

1. Entraînement rotatif électromagnétique, conçu comme un moteur de temple, comprenant un rotor (3), qui comprend un noyau à effet magnétique (31) en forme d'anneau ou de disque, ainsi qu'un stator (2), qui est réalisé sous la forme d'un stator de palier et d'entraînement, avec lequel le rotor (3), dans l'état de fonctionnement, peut être entraîné magnétiquement sans contact autour d'un axe de rotation de consigne, qui définit une direction axiale (A), et avec lequel le rotor (3) peut être supporté magnétiquement sans contact par rapport au stator (2), le rotor (3) étant supporté magnétiquement de manière active dans un plan radial (E) perpendiculaire à la direction axiale (A), et étant stabilisé magnétiquement de manière passive dans la direction axiale (A) ainsi que contre des basculements, le stator (2) présentant une pluralité de noyaux de bobine (25), dont chacun comprend une branche longitudinale (26), qui s'étend dans la direction axiale (A), ainsi qu'une branche transversale (27) disposée dans le plan radial (E), qui s'étend à partir de la branche longitudinale (26) dans une direction radiale, et est limitée par une surface frontale (211), qui est tournée vers le noyau à effet magnétique (31) du rotor (3), et au moins un enroulement concentré (61) étant disposé au niveau de chaque branche longitudinale (26), lequel entoure la branche longitudinale (26) respective, chaque branche longitudinale (26) comprenant une première portion (261) et une deuxième portion (262), qui sont disposées de manière adjacente l'une à l'autre par rapport à la direction axiale (A), la branche transversale (27) étant disposée au niveau de la deuxième portion (262), la surface frontale (211) de la branche transversale (27) présentant une première distance (A) dans la direction radiale par rapport à la première portion (261) de la branche longitudinale (26) associée, et une deuxième distance (A2) dans la direction radiale par rapport à la deuxième portion (262), **caractérisé en ce que** la deuxième distance (A2) est supérieure à la première distance (A1).

2. Entraînement rotatif selon la revendication 1, pour chaque branche longitudinale (26), la première portion (261) et la deuxième portion (262) étant disposées parallèlement l'une à l'autre, et étant reliées l'une à l'autre par une zone de transition (263), dans laquelle la distance radiale par rapport à la surface frontale (211) associée varie de la première distance (A1) à la deuxième distance (A2).

3. Entraînement rotatif selon l'une quelconque des revendications précédentes, le noyau à effet magnétique (31) du rotor (3) présentant une hauteur de rotor (HR), qui est l'étendue maximale du noyau à effet magnétique (31) dans la direction axiale (A), et la hauteur de rotor (HR) étant supérieure à une hauteur de pôle de stator (HS), qui est définie par l'étendue maximale des surfaces frontales (211) des branches transversales dans la direction axiale (A).

4. Entraînement rotatif selon l'une quelconque des revendications précédentes, la deuxième distance (A2) étant supérieure à la moitié d'une hauteur de pôle de stator (HS), qui est définie par l'étendue maximale des surfaces frontales (211) des branches transversales (27) dans la direction axiale (A).

5. Entraînement rotatif selon l'une quelconque des revendications 3 à 5, le noyau à effet magnétique (31) comprenant une zone centrale (32), qui est disposée par rapport à la direction axiale (A) entre une première zone de bord (33) et une deuxième zone de bord (34), et qui présente un diamètre de rotor (RZ), la première zone de bord (33) formant une première surface de limitation axiale (311) du noyau à effet magnétique (31), qui présente un premier diamètre de bord (R1), la deuxième zone de bord (34) formant une deuxième surface de limitation axiale (312) du noyau à effet magnétique (31), qui présente un deuxième diamètre de bord (R2), et chaque diamètre de bord (R1, R2) étant inférieur au diamètre de rotor (RZ).

6. Entraînement rotatif selon la revendication 5, la zone centrale (32) présentant une hauteur centrale (HZ), qui est l'étendue de la zone centrale (32) dans la direction axiale (A), la hauteur centrale (HZ) étant égale à la hauteur de pôle de stator (HS).

7. Entraînement rotatif selon l'une quelconque des revendications 5 à 6, le noyau à effet magnétique (31) présentant une surface extérieure, qui ne s'étend ni entre la zone centrale (32) et la première surface de limitation axiale (311) ni entre la zone centrale (32) et la deuxième surface de limitation axiale (312) parallèlement à la direction axiale (A).

8. Entraînement rotatif selon l'une quelconque des revendications précédentes, deux enroulements concentrés (61a, 61b) étant prévus sur chaque branche longitudinale, dont chacun entoure la branche longitudinale (26) respective, et qui sont disposés de manière adjacente l'une à l'autre par rapport à la direction axiale (A).

9. Pompe centrifuge pour débiter un fluide, **caractérisée en ce que** la pompe centrifuge comprend un entraînement rotatif électromagnétique (1), qui est configuré selon l'une quelconque des revendications précédentes, le rotor (3) de l'entraînement rotatif électromagnétique (1) étant configuré comme rotor (3) de la pompe centrifuge.

10. Pompe centrifuge selon la revendication 9, qui comprend une unité de pompe (50) avec un boîtier de pompe (51), qui comprend une entrée (52) et une sortie (53) pour le fluide à débiter, le rotor (3) étant disposé dans le boîtier de pompe (51), et comprenant une pluralité d'ailettes (54) pour débiter le fluide, l'unité de pompe (50) étant configurée de telle sorte que l'unité de pompe (50) peut être insérée dans le stator (2) de telle sorte que le noyau à effet magnétique (31) du rotor (3) est entouré par les surfaces frontales (211) des branches transversales (27).

11. Unité de pompe pour une pompe centrifuge, **caractérisée en ce que** l'unité de pompe (50) est configurée pour une pompe centrifuge (100), qui est configurée selon la revendication 10.

12. Unité de pompe selon la revendication 11, le boîtier de pompe (51) comprenant une partie de base (512) et un couvercle (511), qui sont reliés l'un à l'autre de manière étanche, la sortie (53) du boîtier de pompe (51) étant disposée complètement dans la partie de base (512).

13. Unité de pompe selon l'une quelconque des revendications 11 à 12, le rotor (3) présentant un alésage central (36), qui s'étend dans la direction axiale (A) complètement à travers le rotor (3).

14. Unité de pompe selon l'une quelconque des revendications 11 à 13, qui est configurée pour être reliée de manière amovible au stator (2) de la pompe centrifuge (100) selon la revendication 9.

15. Unité de pompe selon l'une quelconque des revendications 11 à 14, l'unité de pompe (50) étant configurée comme dispositif à usage unique pour l'usage unique.
